# FASCICULE DE BREVET EUROPEEN

(11) **EP 1 334 713 B1**
(45) Date de publication et mention de la délivrance du brevet: **07.12.2005**
(21) Numéro de dépôt: 03290258.7
(22) Date de dépôt: 03.02.2003
(51) Int. Cl.: A61K 7/13

(54) **Composition tinctoriale comprenant une base d'oxydation du type diaminopyrazole, une base d'oxidation cationique et un coupleur**
Färbemittel enthaltend Diaminopyrazole und kationische Verbindungen als Oxidationsmittel und einen Kuppler
Dyeing composition comprising diaminopyrazole and cationic compounds as oxidation bases and a coupling agent

(30) Priorité: 12.02.2002 FR 0201713
(43) Date de publication de la demande: 13.08.2003
(73) Titulaire: L'OREAL, 75008 Paris (FR)
(72) Inventeur: Kravtchenko, Sylvain, 92600 Asnieres (FR); Lagrange, Alain, 77700 Coupvray (FR)
(74) Mandataire: Fevrier, Murielle Françoise E.

(56) Documents cités:
- EP-A- 0 692 245
- WO-A-02/46165
- WO-A-99/03836
- DE-A- 3 843 892
- DE-A- 19 643 059
- FR-A- 2 766 177
- US-B1- 6 270 533

## Description

L'invention a pour objet une composition tinctoriale comprenant dans un milieu approprié pour la teinture une première base d'oxydation du type diaminopyrazole, une deuxième base d'oxydation cationique monobenzènique, dibenzénique ou hétérocyclique et un coupleur.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu des compositions tinctoriales comprenant à titre de base d'oxydation des dérivés diaminopyrazoles. Par exemple, la demande de brevet DE 3843892 décrit des compositions tinctoriales pour la teinture de fibres kératiniques comprenant des dérivés 4,5-diaminopyrazoles pouvant être substitués en position 2 par des radicaux alkyles ou hydroxyalkyles. La demande de brevet EP 692 245 décrit des compositions tinctoriales comprenant des dérivés 4,5-diaminopyrazoles associés à des métaphénylènediamines particulières. La demande de brevet DE 19643059 décrit des compositions tinctoriales associant des dérivés 4,5-diaminopyrazoles avec des coupleurs métaaminophénols et métaphénylènediamine. La demande de brevet DE 19646609 décrit des compositions tinctoriales associant des dérivés 4,5-diaminopyrazoles avec des coupleurs benzoxazines.

Il est de plus connu dans la demande de brevet WO99/03819 et WO99/03836 des compositions tinctoriales comprenant des paraphénylènediamines à groupement cationique aliphatique ou cyclique.

Les nuances obtenues à partir de compositions tinctoriales contenant ce type de base d'oxydation ne sont cependant pas suffisamment puissantes, chromatiques et/ou tenaces.

Le but de la présente invention est de fournir de nouvelles compositions tinctoriales pour la teinture de fibres kératiniques contenant des dérivés diaminopyrazoles ne présentant pas les inconvénients de celles de la technique antérieure. En particulier, le but de la présente invention est de fournir des compositions tinctoriales contenant des dérivés diaminopyrazoles qui sont peu sélectives et particulièrement résistantes, tout en étant capables d'engendrer des colorations intenses dans des nuances variées.

Ce but est atteint avec la présente invention qui a pour objet une composition tinctoriale comprenant, dans un milieu de teinture approprié :
- au moins une base d'oxydation 4,5-diaminopyrazole de formule (I) ou les sels d'addition correspondants 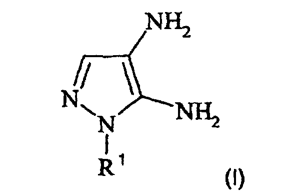
dans laquelle R¹ est un radical alkyle en C₁-C₆ substitué par un ou plusieurs radicaux OR², R² étant un radical alkyle en C₁-C₆ ;
- au moins une base d'oxydation monobenzènique, dibenzénique ou hétérocyclique comportant au moins un groupement cationique Z choisi parmi une chaîne aliphatique portant ou non un cycle saturé ou insaturé ; ledit groupement Z étant directement relié à au moins un cycle benzénique ou à l' hétérocycle de ladite base d'oxydation ou bien relié à au moins une fonction amine portée par ledit cycle benzénique ou ledit hétérocycle ; ou l'un de ses sels d'addition et
- au moins un coupleur.

La composition de la présente invention permet en particulier d'obtenir une coloration des fibres kératiniques chromatique, très puissante, peu sélective et tenace.

Un autre objet de l'invention est l'utilisation de la composition de la présente invention pour la teinture des fibres kératiniques, en particulier les fibres kératiniques humaines telles que les cheveux.

L'invention a aussi pour objet un dispositif et un procédé de teinture mettant en oeuvre la composition de l'invention.

Par la formule (I), il faut comprendre les composés de formule (I) mais aussi toutes les tautomères correspondant à cette formule.

Selon un mode de réalisation particulier, la base d'oxydation 4,5-diaminopyrazole de formule (I) est telle que R¹ représente un radical alkyle en C₁-C₄, de préférence en C₂-C₄ substitué par un radical OR², R² étant un radical alkyle en C₁-C₄, de préférence en C₁-C₂. De préférence, la base d'oxydation de formule (I) est le 4,5-diamino-1-(2'-méthoxyéthyl)-pyrazole ou ses sels d'addition.

Dans le cadre de la présente invention, on entend par alkyle, des radicaux linéaires ou ramifiés par exemple méthyle, éthyle, n-propyle, iso-propyle, butyle, etc. Un radical alcoxy est un radical alk-O, le radical alkyle ayant la définition donnée ci dessus. Halogène désigne de préférence Cl, Br, I, F.

Les bases d'oxydation cationiques conformes à l'invention sont monobenzéniques, dibenzéniques ou hétérocycliques et comportent au moins un groupement cationique Z choisi parmi une chaîne aliphatique portant ou non au moins un cycle saturé ou insaturé ; ledit groupement Z étant directement relié à au moins un cycle benzénique ou à l'hétérocycle de ladite base d'oxydation ou bien relié à au moins une fonction amine portée par ledit cycle benzénique ou ledit hétérocycle ; ou l'un de ses sels d'addition et

Z est choisi de préférence parmi les groupements cationiques insaturés de formules (II) et (III) suivantes, et les groupements cationiques saturés de formule (IV) suivante : 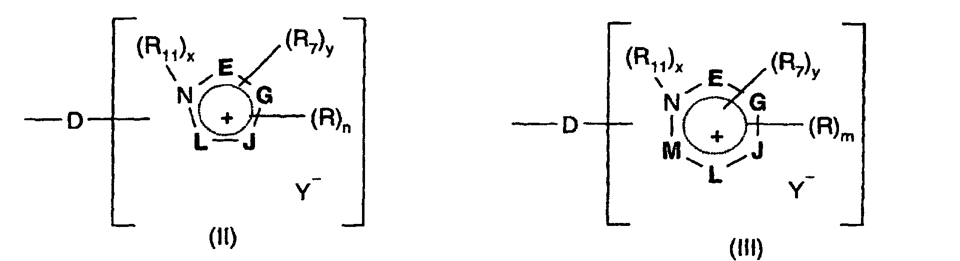 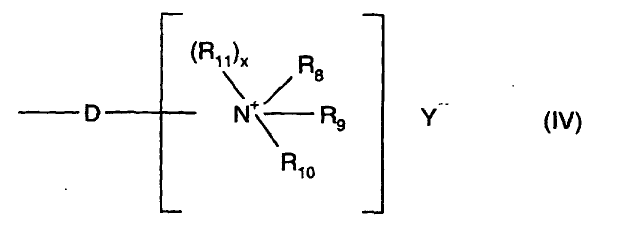 dans lesquelles :
- D est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, représentent un atome de carbone, d'oxygène, de soufre ou d'azote ;
- n est un nombre entier allant de 0 à 4 ;
- m est un nombre entier allant de 0 à 5 ;
- les radicaux R, R₈ à R₁₁, identiques ou différents, représentent un atome d'halogène, un radical hydroxyle, un groupe amino, un groupe cyano, un groupe amido, SO₃X un aryle, un radical Z₁, OZ₁, NHZ₁, NZ₁Z₂ ; deux des radicaux R₈ à R₁₀ peuvent former un hétérocycle saturé à 5 ou 6 chaînons pouvant être interrompue par un ou plusieurs hétéroatomes et pouvant comporter un ou plusieurs substituants choisis parmi les groupements hydroxyle, amino, halogène, cyano, amido, CO₂X, un radical alkyle en C₁-C₆, un radical alcoxy en C₁-C₆, un radical monoalkylamino en C₁-C₆, un radical dialkylamino en C₁-C₆ ;
- Z₁ et Z₂, identiques ou différents, représentent une chaîne hydrocarbonée en C₁-C₁₄, linéaire ou ramifiée, pouvant être interrompue par un ou plusieurs hétéroatomes et pouvant comporter un ou plusieurs substituants choisis parmi les groupements hydroxyle, amino, amido, halogène, cyano, CO₂X, un radical alkyle en C₁-C₆, un radical alcoxy en C₁-C₆, un radical monoalkylamino en C₁-C₆, un radical dialkylamino en C₁-C₆ ;
- X désigne hydrogène, un reste de métal alcalin ou alcalino-terreux, l'ion ammonium, un radical alkyle en C₁-C₆ ou un reste issu d'une amine organique ;
- Y⁻ représente un anion monovalent ou divalent issu d'un acide organique ou minéral et est de préférence choisi parmi un atome d'halogène tel que le chlore, le brome, le fluor ou l'iode, un hydrogènosulfate, ou un alkyl(C₁-C₆)sulfate tel que par exemple un méthylsulfate ou un éthylsulfate.
- x et y sont des nombres entiers égaux à 0 ou 1 ; avec les conditions suivantes :
dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
   1) lorsque les sommets E, G, J et L représentent simultanément un atome de carbone, et que le radical R₇ est porté par l'atome d'azote du cycle insaturé ; ou bien
   2) lorsqu'au moins un des sommets E, G, J et L représente un atome d'azote sur lequel le radical R₇ est fixé ;
   dans les groupements cationiques insaturés de formule (III) :
   - lorsque x = 0, le bras de liaison D est rattaché à l'atome d'azote,
   - lorsque x = 1, le bras de liaison D est rattaché à l'un des sommets E, G, J, L ou M,
   - y ne peut prendre la valeur 1 que lorsqu'au moins un des sommets E, G, J, L et M représente un atome divalent, et que le radical R₇ est porté par l'atome d'azote du cycle insaturé ;
   dans les groupements cationiques de formule (IV) :
   - lorsque x = 0, alors le bras de liaison est rattaché à l'atome d'azote portant les radicaux R₈ à R₁₀,
   - lorsque x = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé.

Parmi les cycles insaturés des groupements Z de formule (II) ci-dessus, on peut notamment citer à titre d'exemple les cycles pyrrolique, imidazolique, pyrazolique, oxazolique, thiazolique et triazolique.

Parmi les cycles insaturés des groupements Z de formule (III) ci-dessus, on peut notamment citer à titre d'exemple les cycles pyridinique, pyrimidinique, pyrazinique, oxazinique et triazinique.

Parmi les cycles saturés des groupements Z de formule (IV) ci-dessus, on peut notamment citer à titre d'exemple les cycles pyrrolidine, pipéridine, pipérazine, morpholine.

De préférence, D représente une chaine alkylène comprenant de 1 à 10 atomes de carbone.

Les bases d'oxydation monobenzéniques, dibenzéniques ou hétérocycliques peu^vent être substituées ou non sur les cycles monobenzéniques, dibenzéniques ou hétérocycliques.

Les bases d'oxydation cationiques conformes à l'invention sont de préférence choisies parmi :
(i) les paraphénylènediamines ou les para-aminophénols monobenzéniques telles que celles décrites dans les demandes EP968171 et EP928289 (faisant partie intégrante du contenu de la description) ;
(ii) les paraphénylènediamines ou les para-aminophénols dibenzéniques telles que celles décrites dans la demande EP932602 (faisant partie intégrante du contenu de la description) ;
(iii) les orthophénylènediamines monobenzéniques telles que celles décrites dans les demandes EP983996 et EP984007 (faisant partie intégrante du contenu de la description) ;
(iv) les orthophénylènediamines dibenzéniques telles que celles décrites dans la demande EP984006 (faisant partie intégrante du contenu de la description) ;
(v) les bases hétérocycliques du type pyrazole telles que celles décrites dans la demande EP1147090 (faisant partie intégrante du contenu de la description) ;
(vi) les bases hétérocycliques du type pyrazolo-[1,5-a]-pyrimidine telles que celles décrites dans la demande EP1147109 (faisant partie intégrante du contenu de la description).

Parmi les paraphénylènediamines ou les para-aminophénols monobenzéniques à groupement cationique, on peut citer les composés suivants :
- le chlorure de [2-(2,5-diamino-phénoxy)-éthyl]-diéthyl-méthyl-ammonium ;
- le dichlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [4-(4-amino-phénylamino)-pentyl]-diéthyl-(2-hydroxyéthyl)-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [2-(4-amino-phénylamino)-propyl]-triméthyl-ammonium ;
- le chlorure de [4-(4-amino-2-méthyl-phénylamino)-pentyl]-diéthyl-méthyl-ammonium ;
- le chlorure de [4-(4-amino-3-méthyl-phénylamino)-pentyl]-diéthyl-méthyl-ammonium ;
- le chlorure de 1-{[5-amino-2-(2-hydroxyéthylamino)-phénylcarbamoyl]-méthyl}-1,4-diméthyl-pipérazin-1-ium ;
- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol- 1-ium;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthyl-amino]-éthyl}-3-méthyl-3H-imidazol-1-ium ;
- le dichlorure de N,N-bis-[2-(3-méthyl-3H-imidazol-1-ium)-éthyl]-4-amino-aniline ;
- le chlorure de 3-[2-(4-amino-phénylamino)-butyl]-1-méthyl-3H-imidazol-1-ium ;
- le chlorure de 1-{[5-amino-2-(2-hydroxy-éthylamino)-phénylcabamoyl]-méthyl}-3-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium ;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure 4-[(2,5-diamino-phénylcarbamoyl)-méthyl]-1,3-diméthyl-3H-imidazol-1-ium ;
- le chlorure de 4-{2-[2-(2-amino-5-hydroxy-phényl)-acétylamino]-éthyl}-1,3-diméthyl-3H-imidazol-1-ium ;
- le chlorure de 4-[(5-amino-2-hydroxy-benzylcarbamoyl)-méthyl]-1,3-diméthyl-3H-imidazol-1-ium ; et leurs sels d'addition.

Parmi ces composés, on préfère plus particulièrement :
- le chlorure de [2-(2,5-diamino-phénoxy)-éthyl]-diéthyl-méthyl-ammonium ;
- le chlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [4-(4-amino-phénylamino)-pentyl]-diéthyl-(2-hydroxyéthyl)-ammonium ;
- le chlorure de 3-[3-(4-amino-phénylamino)-propyl]-1-méthyl-3H-imidazol-1-ium ;
- le chlorure de1-[3-(2,5-diamino-phénoxy)-propyl]-3-méthyl-3H-imidazol-1-ium ;
- le chlorure, de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 3-[3-(4-amino-2-fluoro-phénylamino)-propyl]-1-méthyl-3H-imidazol-1-ium ;
- le chlorure de 3-[3-(4-amino-2-cyano-phénylamino)-propyl]-1-méthyl-3H-imidazol-1-ium et leurs sels d'addition.

Parmi les paraphénylènediamines ou les para-aminophénols dibenzéniques à groupement cationique, on peut citer les composés suivants :
- le dichlorure de 1,3-bis-1{3{3'[(4"-amino-3"-méthyl-aniline)-N-propyl]}-3H imidazol-1-ium}-propane ;
- le dichlorure de 1,3-bis-1{3{3'[(4"-amino-2"-méthyl-aniline)-N-propyl]}-3H-imidazol-1-ium}-propane ;
- le dichlorure de 1,3-bis-1{3{3'[(4"-amino-aniline)-N-propyl]}-3H-imidazol-1-ium}-propane ;
- le dichlorure de 1,3-bis-1{3{3'[(4"-amino-aniline)-N-propyl]}-3H-imidazol-1-ium}-2-propanol ;
- le dibromure de N₁,N₃-bis-[3-N(4'-amino-aniline)-propyl]-1,1,3,3-tétraméthyl-diammonium 1-3-propane ;
- le dichlorure de 1,4-bis-1{3[3-(2,5-diamino-phénoxy)-propyl]-3H-imidazol-1-ium}- butane ;
- le monochlorure de 1,3-bis-[3-(2,5-diamino-phénoxy)-propyl]-3H-imidazol-1-ium ;
- le dibromure de N₁,N₄-bis-[3-N-méthyl-N-(4'-amino-aniline)-éthyl]-1,1,4,4-tétraméthyl-diammonium 1-3-propane ;
- le dichlorure de 1,4-bis-1-[3-(5-amino-2-hydroxy-benzyl)-3H-imidazol-1ium]-butane ;
- le dibromure de 1,3-bis-{[2-(4-amino-aniline)-propyl]-1,1,3,3-tétraméthyl-diammonium-propane ;
- le dichlorure de 1,3-bis-{[4-(4-amino-aniline)-pentyl]-1,1,3,3-tétraméthyl-diammonium-propane ;
- le monochlorure de [4-(4-amino-phénylamino)-pentyl]-(5-amino-2-hydroxy-benzyl)-diéthyl-ammonium ;
- le monochlorure de [2-(4-amino-phénylamino)-propyl]-(5-amino-2-hydroxy-benzyl)-diméthyl-ammonium ;
- le dichlorure de 1,3-bis-1-{3-[3-(2,5-diamino-phénoxy)-propyl]-3H-imidazol-1-ium}-propane ;
- le dichlorure de 1,3-bis-1{3{3'[(4"-amino-aniline)-N-propyl]}-3H-imidazol-1-ium}-propane ;
- le dichlorure de 1,3-bis-1{4{4'(4-[3-(4"-amino-phényiamino)-propyl] }-1,3-diméthyl-3H-imidazol-1-ium}-propane,
- le dichlorure de 1,3-bis-1{4{4'(4-[3-(4"-amino-2"-méthyl-aniline)-propyl] }-1,3-diméthyl-3H-imidazol-1-ium}-propane ;
- le monochlorure de 4-[2-(2,5-diamino-phénoxy)-éthyl]-3-[3-(2,5-diamino-phénoxy)-propyl]-1-méthyl-3-imidazol-1-ium ;
- le monochlorure de 4-[2-(2,5-diamino-phénoxy)-éthyl]-1-[3-(2,5-diamino-phénoxy)-propyl]-3-méthyl-3-imidazol-1-ium ;
et leurs sels d'addition.

Parmi ces composés, on préfère plus particulièrement :
- le dichlorure de 1,3-bis-1-{3-{3'-[(4"-amino-aniline)-N-propyl]}-3H-imidazol-1-ium}-propane ;
- le monochlorure de 1,3-bis-[3-(2,5-diamino-phénoxy)-propyl]-3H-imidazol-1-ium ;
- le dibromure de N₁,N₄-bis-[3-N-méthyl-N-(4'-amino-aniline)-éthyl]-1,1,4,4-tétraméthyl-diammonium 1-3-propane ;
- le dichlorure de 1,4-bis-1 [3-(5-amino-2-hydroxy-benzyl)-3H-imidazol-1-ium]-butane ;
- le dichlorure de 1,3-bis-1{3{3'[(4"-amino-aniline)-N-propyl]}-3H-imidazol-1-ium}-propane ;
et leurs sels d'addition.

Parmi les orthophénylènediamines monobenzéniques à groupement cationique, on peut citer les composés suivants :
- le monochlorure de {2-[2-amino-phénylamino]-éthyl}-triméthyl-ammonium ;
- le monochlorure de [2-(2-amino-5-chloro-phénylamino)-éthyo-triméthyl-ammonium ;
- le monochlorure de [2-(2-amino-6-chloro-phénylamino)-éthyl]-triméthyl-ammonium ;
- le monochlorure de [2-(2-amino-4-chloro-phénylamino)-éthyl]-triméthyl-ammonium ;
- le monochlorure de {2-[2-amino-4-chloro-5-(2-hydroxyéthoxy)-phénylamino]-éthyl}-triméthyl-ammonium ;
- le monochlorure de [2-(2-amino-5-méthoxy-phénylamino)-éthyl]-triméthyl-ammonium ;
- le monobromure de [2-(2-amino-phénylamino)-éthyl]-(2-hydroxyethyl)-diméthyl-ammonium ;
- le monochlorure de 4-[2-(2-amino-phénylamino)-éthyl]-4-méthyl-morpholin-4-ium ;
- le monochlorure de 1-[2-(2-amino-phénylamino)-éthyl]-1-éthyl-pipéridinium ;
- le monochlorure de 1-[2-(2-amino-phénylamino)-éthyl]-1,4-diméthyl-pipérazin-1-ium ;
- le dichlorure de 4-[2-(1-méthyl-pipéridinium)-éthoxy]-N2-[2-(1-méthyl-pipéridinium)-éthyl]-benzène-1,2-diamine ;
- le monochlorure de 1-[2-(2-amino-5-méthylsulfanyl-phénylamino)-éthyl]-1-méthyl-pipéridinium ;
- le monochlorure de 1-[2-(2-amino-phénylamino)-éthyl]-1-méthyl-pyrrolidinium ;
- le monochlorure de [3-(2-amino-phénylamino)-propyl]-diéthyl-méthyl-ammonium ;
- le dichlorure de N,N'-bis-[2-(1-méthyl-pipéridinium)-éthyl]-benzène-1,2-diamine ;
- le monochlorure de [2-(2-amino-4-méthyl-phénylamino)-éthyl]-triméthyl-
- le monochlorure de 3-[3-(2-amino-phénylamino)-propyl]-1-méthyl-3H-imidazol-1-ium ;
- le monochlorure de 3-[2-(2-amino-phénylamino)-éthyl]-1-méthyl-3H-imidazol-1-ium ;
- le dichlorure de 4-[2-(1-méthyl-3H-imidazol-1-ium)-éthoxy]-N2-[2-(1-méthyl-3H-imidazol-1-ium)-éthyl]-benzène-1,2-diamine ;
- le monochlorure de 3-[2-(2-amino-4-méthyl-phénylamino)-éthyl]-1-éthyl-3H-imidazol-1-ium ;
- le dichlorure de 3-[3-(2-amino-phénylamino)-propyl]-1-(3-triméthyl-ammonium-2-hydroxy-propyl)-3H-imidazol-1-ium ;
- le monobromure de 3-[3-(2-amino-phénylamino)-propyl]-1-(2-hydroxy-éthyl)-3H-imidazol-1-ium
- le monochlorure de 3-{[2-(2-amino-phénylamino)-éthylcarbamoyl]-méthyl}-1-méthyl-3H-imidazol-1-ium ;
- le monochlorure de 1-[2-(2-amino-4-chloro-phénylamino)-éthyl]-pyridinium ;
- le monochlorure de 3-[2-(2-amino-5-méthoxy-phénylamino)-éthyl]-1-méthyl-3H-imidazol-1-ium ;
- le monochlorure de 3-[2-(2-amino-5-méthylsulfanyl-phénylamino)-éthyl]-1-méthyl-3H-imidazol-1-ium ;
et leurs sels d'addition.

Parmi ces composés, on prèfère tout particulièrement :
- le chlorure de {2-[2-amino-phénylamino]-éthyl}-triméthyl-ammonium ;
- le chlorure de 3-[3-(2-amino-phénylamino)-propyl]-1-méthyl-3H-imidazol-1-ium
et leurs sels d'addition.

Parmi les orthophénylènediamines dibenzéniques à groupement cationique, on peut citer les composés choisis parmi :
- le dibromure de 1,3-bis-{3-{3-[(2-amino-aniline)-N-propyl]}-3H-imidazol-1-ium}-propane,
- le dibromure de N₁,N₃-bis-[3-N-(2-amino-aniline)-propyl]-1,1,3,3-tétraméthyl-diammonium 1-3-propane,
- le dichlorure de 1,4-bis-{3-{2-[(2-amino-aniline)-N-éthyl]}-3H-imidazol-1-ium}-butane,
- le monochlorure de 1-[2-(2-amino-aniline)-éthyl]-3-[3-(2-amino-aniline)-propyl]-3H-imidazol-1-ium, et leurs sels d'addition.

Parmi ces composés, on préfère tout particulièrement :
- le dibromure de 1,3-bis-1-{3-{3-[(2-amino-aniline)-N-propyl]}-3H-imidazol-1-ium}-propane,
le dibromure de N1,N3-bis-[3-N-(2-amino-aniline)-propyl]-1,1,3,3-tétraméthyl-diammonium 1-3-propane, et leurs sels d'addition.

Parmi les bases hétérocycliques cationiques du type pyrazole, on peut citer :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-pyrazol-1-yl)-éthyl]-1-méthyl-3H-imidazol-1-ium;
- le chlorure de [2-(4,5-diamino-3-méthyl-pyrazol-1-yl)-éthyl]-triméthylammonium ;
- le chlorure de [2-(4,5-diamino-pyrazol-1-yl)-éthyl]-triméthyl-ammonium ;
- le chlorure de [2-(4-amino-5-hydroxy-pyrazol-1-yl)-éthyl]-triméthyl-ammonium ;
- le chlorure de [2-(4-amino-5-hydroxy-3-méthyl-pyrazol-1-yl)-éthyl]-triméthylammonium ;
- le chlorure de 3-[2-(4-amino-5-hydroxy-3-méthyl-pyrazol-1-yl)-éthyl]-1-méthyl-3H-imidazol-1-ium ;
- le chlorure de 3-[2-(4-amino-5-hydroxy-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium,
et leurs sels d'addition et leurs possibles formes tautomères.

Parmi ces composés, on préfère plus particulièrement :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium ;
et leurs sels d'addition et leurs possibles formes tautomères.

Parmi les bases hétérocycliques du type pyrazolo-[1,5-a]-pyrimidine, on peut citer :
- le monobromure de 1-[3-(3-amino-5,6-diméthyl-pyrazolo-[1,5-a]-pyrimidin-7-ylamino)-propyl]-3-méthyl- 3H-imidazol-1-ium ,
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le chlorure de 3-[(3-amino-pyrazolo[1,5-a]pyrimidin-7-ylcarbamoyl)-méthyl]-1-méthyl-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le méthyl sulfate de 2-[(3-amino-pyrazolo[1,5-a]pyrimidin-7-ylamino)-méthyl]-1,3-diméthyl-3H-imidazol-1-ium,
- le méthyl sulfate de 3-[(3-amino-pyrazolo[1,5-a]pyrimidin-7-ylamino)-méthyl]-1-méthyl-pyridinium,
- le méthyl sulfate de 3-[(3-amino-pyrazolo[1,5-a]pyrimidin-7-ylamino)-méthyl]-1-méthyl-pyridinium,
- le méthyl sulfate de 2-(3,7-diamino-5-méthyl-pyrazolo[1,5-a]pyrimidin-6-ylméthyl)-1,3-diméthyl-3H-imidazol-1-ium,
- le méthyl sulfate de 2-(3-amino-7-hydroxy-5-méthyl-pyrazolo[1,5-a]pyrimidin-6-ylméthyl)-1,3-diméthyl-3H-imidazol-1-ium,
- le méthyl sulfate de 2-(3,7-diamino-pyrazolo[1,5-a]pyrimidin-2-yl)-1-méthyl-pyridinium,
- le chlorure de [3-(3-amino-5-méthyl-pyrazolo[1,5-a]pyrimidin-7-ylamino)-propyl]-triméthyl-ammonium,
- le méthyl sulfate de [3-(3-amino-5-méthyl-pyrazolo[1,5-a]pyrimidin-7-ylamino)-propyl]-triméthyl-ammonium,
- le chlorure de 1-[3-(3-amino-5-méthyl-pyrazolo[1,5-a]pyrimidin-7-ylamino)-propyl]-1-méthyl-pipéridinium,
- le méthyl sulfate de 1-[3-(3-amino-5-méthyl-pyrazolo[1,5-a]pyrimidin-7-ylamino)-propyl]-1-méthyl-pipéridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition.

Parmi ces composés, on préfère plus particulièrement :
- le monobromure de 1-[3-(3-amino-5,6-diméthyl-pyrazolo-[1,5-a]-pyrimidin-7-ylamino)-propyl]-3-méthyl- 3H-imidazol-1-ium ,
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le chlorure de 3-(3-amino-7-hydroxy-5-méthyl-pyrazolo[1,5-a]pyrimidin-6-ylméthyl)-1-méthyl-pyridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition.

Conformément à la présente invention, on utilisera plus particulièrement les bases cationiques du type paraphénylènediamine monobenzénique.

Le coupleur utile dans la composition de la présente invention peut être n'importe quel coupleur classiquement utilisé dans le domaine de la coloration. Ce coupleur peut être choisi parmi les métaphénylènediamines, les méta-aminophénols, les métadiphénols, les coupleurs naphtaléniques, les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

La composition de la présente invention peut en outre comprendre une ou plusieurs bases d'oxydation additionnelles non cationiques classiquement utilisées en teinture d'oxydation autres que celles décrites précédemment. A titre d'exemple, ces bases d'oxydation additionnelles sont choisies parmi les paraphénylènediamines autres que celles décrites précédemment, les bis-phénylalkylènediamines, les para-aminophénols, les ortho-aminophénols, les bases hétérocycliques autres que celles décrites précédemment et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 2-thiényl paraphénylènediamine, le 2-β hydroxyéthylamino 5-amino toluène, et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.
Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques autres que celles utiles dans la présente invention, on peut citer à titre d'exemple, les dérivés pyridiniques, les dérivés pyrimidiniques et les dérivés pyrazoliques autres que ceux de l'invention.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide ou une base.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine; la 2-acétylamino pyrazolo-[1,5-a] pyridin-3-ylamine ; la 2-morpholin-4-yl-pyrazolo[1,5-a]pyridin-3-ylamine; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol; la 3,6-diamino-pyrazolo[1,5-a]pyridine ; la 3,4-diamino-pyrazolo[1,5-a]pyridine; la pyrazolo[1,5-a]pyridine-3,7-diamine ; la 7-morpholin-4-yl-pyrazolo[1,5-a]pyridin-3-ylamine; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ainsi que leurs d'addition avec un acide ou avec une base.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 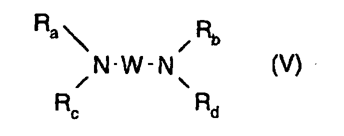 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Selon le procédé de teinture de la présente invention, on applique sur les fibres la composition selon la présente invention, et on révèle la couleur à l'aide d'un agent oxydant. La couleur peut être révélée à pH acide, neutre ou alcalin et l'agent oxydant peut être mélangé à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment pour la composition de l'invention.

Le pH de la composition oxydante renfermant l'agent oxydant est tel qu'après mélange avec la composition tinctoriale, le pH de la composition résultante appliquée sur les fibres kératiniques varie entre 3 et 12 environ, et préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment contient la composition tinctoriale de l'invention définie ci-dessus et un deuxième compartiment contient une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Les composés diaminopyrazoles utiles dans la composition de la présente invention sont des composés connus qui peuvent être obtenus à partir des procédés de préparation généraux connus de l'homme du métier. Par exemple, l'approche synthétique montrée ci-dessous est décrite dans la littérature jusqu'à l'intermédiaire (2) (J. H. P. Juffermanns, C. L ; Habraken ; J. Org. Chem., 1986, 51, 4656 ; Klebe et al.; Synthesis, 1973, 294 ; R. Hüttel, F. Büchele ; Chem. Ber.; 1955, 88, 1586.). Dans le cas présente, le passage du composé 3 au composé 2 s'effectue au moyen d'un mélange NH₃/EtOH.

L'alkylation et l'amination pour arriver aux composés de formule (1) selon l'invention sont mentionnées dans le document DE 42 34 885. 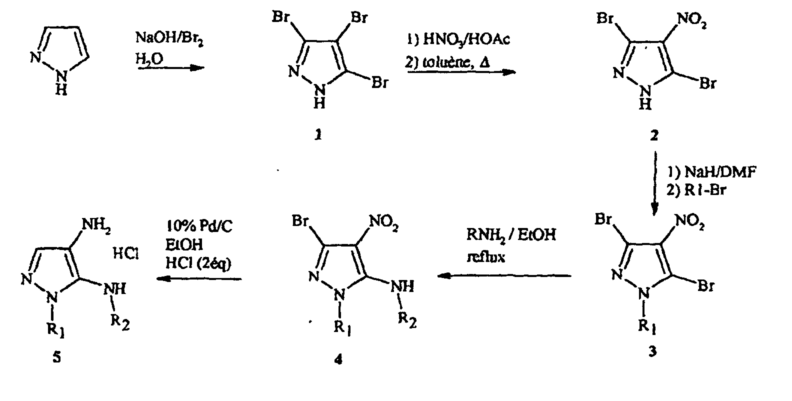 Les exemples qui suivent servent à illustrer l'invention sans toutefois présenter un caractère limitatif.

### EXEMPLES

### Exemple 1. Synthèse du 4,5-Diamino-1-(2'-méthoxyéthyl)-pyrazole dichlorhydrate

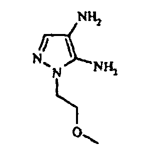

Un mélange de 5-benzylamino-3-bromo-1-(2'-méthoxyéthyl)-4-nitropyrazole (4 g, 2,8 mmoles) dans l'éthanol (500 ml) contenant un catalyseur de 10%Pd/C (Johnson-Mattey Type 487, poids sec 0,5 g) et 36% d'acide chlorhydrique (0.57 g, 5,6 mmoles) est hydrogéné dans un Parr Autoclave (1 l) à 1 MPa pendant 1h. Le catalyseur est ensuite enlevé par filtration, lavé à l'éthanol et le filtrat évaporé sous pression réduite. On obtient ainsi un solide orange brut (2,8 g) qui a été trituré dans l'AcOEt (20 ml) pendant 1 h. Le solide est ensuite filtré et lavé avec de l'AcOEt à froid (20 ml) puis séché sous vide afin de donner la 4,5-Diamino-1-(2'-méthoxyéthyl)-pyrazole sous forme de solide beige (0,7 g, 27%).
HPLC (pureté) : 99,5%
P.F. : 168,1-173,0°C
RMN : ¹H (400 MHz, d⁶-DMSO) : 7,34 (1 H, s, NHₐᵣₒₘ), 5,18 (1 H, S_{large}, NH), 4,09 (2 H, t, J = 5.5 Hz, CH₂N), 3,61 (2 H, t, J = 5.5 Hz, CH₂O), 3,23 (3 H, s, OCH₃).

### Exemple 2. Composition tinctoriale contenant du 4,5-Diamino-1-(2'-méthoxyéthyl)-pyrazole dichlorhydrate

On a préparé la composition tinctoriale suivante:

| **Exemple** | **2** |
|---|---|
| 4,5-Diamino-1-(2'-méthoxyéthyl)-pyrazole, 2HCl | 6. 10⁻³ moles |
| Monochlorure de 1,3-bis-[3-(2,5-diaminophénoxy)-propyl]-3H-imidazol-1-ium tétrachlorhydrate monohydrate (base d'oxydation) | , 2. 10⁻³ moles |
| 2-méthyl-5-aminophénol (coupleur) | 8.10⁻³ moles |
| Support de teinture | (*) |
| Eau déminéralisée q.s.p. | 100 g |

| (*) Support de teinture | |
|---|---|
| Alcool bonzylique | 2 g |
| Polyéthylène glycol 8 OE | 3 g |
| Ethanol | 18 g |
| Alkyl (C8-C10) polyglucoside en solution aqueuse à 60% de matière active tamponné par du citrate d'ammonium vendu sous la dénomination ORAMIX CG110 par SEPPIC | 5 g en l'état |
| Ammoniaque à 20% de NH3 | 10 g |
| Métabisulfite de sodium | 0,205 g |
| Séquestrant | q.s. |

Au moment de l'emploi, la composition est mélangée avec un poids égal d'eau oxygénée à 20 volumes (6% en poids).

Le mélange obtenu est appliqué sur des mèches de cheveux gris à 90 % de blancs naturels et permanentés à raison de 10g pour 1g de cheveux. Après 30 min de pose, les mèches sont rinçées, lavées avec un shampooing standard, rinçées à nouveau puis séchées.

L'évaluation des mèches est effectuée de manière visuelle. On obtient ainsi une coloration acajou cuivrée intense.

## Revendications

1. Composition tinctoriale comprenant, dans un milieu de teinture approprié :
- au moins une base d'oxydation 4,5-diaminopyrazole de formule (I) ou les sels d'addition correspondants 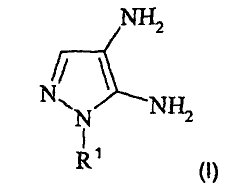
dans laquelle R¹ est un radical alkyle en C₁-C₆ substitué par un ou plusieurs radicaux OR², R² étant un radical alkyle en C₁-C₆ ;
- au moins une base d'oxydation monobenzènique, dibenzénique ou hétérocyclique comportant au moins un groupement cationique Z choisi parmi une chaîne aliphatique portant ou non un cycle saturé ou insaturé ; ledit groupement Z étant directement relié à au moins un cycle benzénique ou à l' hétérocycle de ladite base d'oxydation ou bien relié à au moins une fonction amine portée par ledit cycle benzénique ou ledit hétérocycle ; ou l'un de ses sels d'addition et
- au moins un coupleur.

2. Composition selon la revendication 1 dans laquelle la base d'oxydation de formule (I) est telle que R¹ représente un radical alkyle en C₁-C₄ substitué par un radical OR², R₂ étant un radical alkyle en C₁-C₄.

3. Composition selon la revendication 2, dans laquelle la base d'oxydation de formule (I) est le 4,5-diamino-1-(2'-méthoxyéthyl)-pyrazole.

4. Composition selon l'une quelconque des revendications 1 à 3, dans laquelle Z est choisi parmi les groupements cationiques insaturés de formules (II) et (III) suivantes, et les groupements cationiques saturés de formule (IV) suivante : 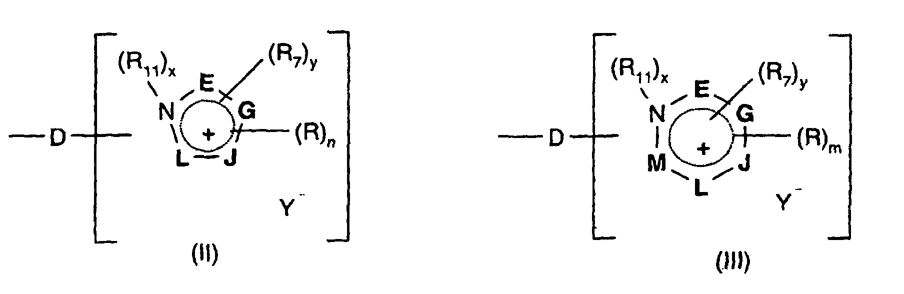 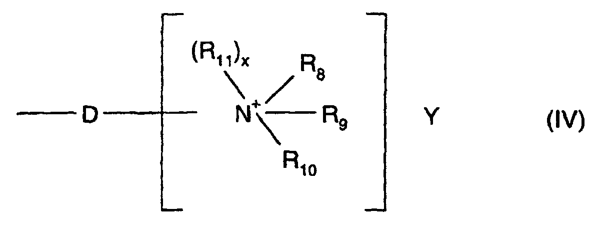 dans lesquelles :
- D est un bras de liaison qui représente une chaîne alkylène comportant de préférence de 1 à 14 atomes de carbone, linéaire ou ramifiée pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆, et pouvant porter une ou plusieurs fonctions cétone ;
- les sommets E, G, J, L et M, identiques ou différents, représentent un atome de carbone, d'oxygène, de soufre ou d'azote ;
- n est un nombre entier allant de 0 à 4 ;
- m est un nombre entier allant de 0 à 5 ;
- les radicaux R, R₈ à R₁₁, identiques ou différents, représentent un atome d'halogène, un radical hydroxyle, un groupe amino, un groupe cyano, un groupe amido, SO₃X un aryle, un radical Z₁, OZ₁, NHZ₁, NZ₁Z₂; deux des radicaux R₈ à R₁₀ peuvent former un hétérocycle saturé à 5 ou 6 chaînons pouvant être interrompue par un ou plusieurs hétéroatomes et pouvant comporter un ou plusieurs substituants choisis parmi les groupements hydroxyle, amino, halogène, cyano, amido, CO₂X, un radical alkyle en C₁-C₆, un radical alcoxy en C₁-C₆, un radical monoalkylamino en C₁-C₆, un radical dialkylamino en C₁-C₆ ;
- Z₁ et Z₂, identiques ou différents, représentent une chaîne hydrocarbonée en C₁-C₁₄, linéaire ou ramifiée, pouvant être interrompue par un ou plusieurs hétéroatomes et pouvant comporter un ou plusieurs substituants choisis parmi les groupements hydroxyle, amino, amido, halogène, cyano, CO₂X, un radical alkyle en C₁-C₆, un radical alcoxy en C₁-C₆, un radical monoalkylamino en C₁-C₆, un radical dialkylamino en C₁-C₆ ;
- X désigne hydrogène, un reste de métal alcalin ou alcalino-terreux, l'ion ammonium, un radical alkyle en C₁-C₆ ou un reste issu d'une amine organique ;
- Y représente un anion monovalent ou divalent issu d'un acide organique ou minéral et est de préférence choisi parmi un atome d'halogène tel que le chlore, le brome, le fluor ou l'iode, un hydrogènosulfate, ou un alkyl(C₁-C₆)sulfate tel que par exemple un méthylsulfate ou un éthylsulfate.
- x et y sont des nombres entiers égaux à 0 ou 1 ; avec les conditions suivantes :
dans les groupements cationiques insaturés de formule (II) :
- lorsque x = 0, le bras de liaison D est rattaché à l'atome d'azote,
- lorsque x = 1, le bras de liaison D est rattaché à l'un des sommets E, G, J ou L,
- y ne peut prendre la valeur 1 que :
1) lorsque les sommets E, G, J et L représentent simultanément un atome de carbone, et que le radical R₇ est porté par l'atome d'azote du cycle insaturé ; ou bien
2) lorsqu'au moins un des sommets E, G, J et L représente un atome d'azote sur lequel le radical R₇ est fixé ;
dans les groupements cationiques insaturés de formule (III) :
- lorsque x = 0, le bras de liaison D est rattaché à l'atome d'azote,
- lorsque x = 1, le bras de liaison D est rattaché à l'un des sommets E, G, J, L ou M,
- y ne peut prendre la valeur 1 que lorsqu'au moins un des sommets E, G, J, L et M représente un atome divalent, et que le radical R₇ est porté par l'atome d'azote du cycle insaturé ;
dans les groupements cationiques de formule (IV) :
- lorsque x = 0, alors le bras de liaison est rattaché à l'atome d'azote portant les radicaux R₈ à R₁₀,
- lorsque x = 1, alors deux des radicaux R₈ à R₁₀ forment conjointement avec l'atome d'azote auquel ils sont rattachés un cycle saturé à 5 ou 6 chaînons tel que défini précédemment, et le bras de liaison D est porté par un atome de carbone dudit cycle saturé.

5. Composition selon la revendication 4, dans laquelle les cycles insaturés Z de formule (II) sont choisis parmi les cycles pyrrolique, imidazolique, pyrazolique, oxazolique, thiazolique et triazolique.

6. Composition selon la revendication 4, dans laquelle les cycles insaturés Z de formule (III) sont choisis parmi les cycles pyridinique, pyrimidinique, pyrazinique, oxazinique et triazinique.

7. Composition selon la revendication 4, dans laquelle les cycles saturés Z de formule (IV) sont choisis parmi les cycles pyrrolidine, pipéridine, pipérazine et morpholine.

8. Composition selon l'une quelconque des revendications 1 à 7, dans laquelle les bases d'oxydation cationiques sont choisies parmi :
(i) les paraphénylènediamines ou les para-aminophénols monobenzéniques ;
(ii) les paraphénylènediamines ou les para-aminophénols dibenzéniques ;
(iii) les orthophénylènediamines monobenzéniques ;
(iv) les orthophénylènediamines dibenzéniques ;
(v) les bases hétérocycliques du type pyrazole ;
(vi) les bases hétérocycliques du type pyrazolo-[1,5-a]-pyrimidine.

9. Composition selon la revendication 8, où les paraphénylènediamines ou les para-aminophénols monobenzéniques à groupement cationique sont choisies parmi :
- le chlorure de [2-(2,5-diamino-phénoxy)-éthyl]-diéthyl-méthyl-ammonium ;
- le dichlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [4-(4-amino-phénylamino)-pentyl]-diéthyl-(2-hydroxyéthyl)-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [2-(4-amino-phénylamino)-propyl]-triméthyl-ammonium ;
- le chlorure de [4-(4-amino-2-méthyl-phénylamino)-pentyl]-diéthyl-méthyl-ammonium ;
- le chlorure de [4-(4-amino-3-méthyl-phénylamino)-pentyl]-diéthyl-méthyl-ammonium ;
- le chlorure de 1-{[5-amino-2-(2-hydroxyéthylamino)-phénylcarbamoyl]-méthyl}-1,4-diméthyl-pipérazin-1-ium ;
- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 3-[3-(4-amino-2-fluoro-phénylamino)-propyl]-1-méthyl-3H-imidazol- 1-ium ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthyl-amino]-éthyl}-3-méthyl-3H-imidazol-1-ium ;
- le dichlorure de N,N-bis-[2-(3-méthyl-3H-imidazol-1-ium)-éthyl]-4-aminoaniline ;
- le chlorure de 3-[2-(4-amino-phénylamino)-butyl]-1-méthyl-3H-imidazol-1-ium ;
- le chlorure de 1-{[5-amino-2-(2-hydroxy-éthylamino)-phénylcarbamoyl]-méthyl}-3-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium ;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure 4-[(2,5-diamino-phénylcarbamoyl)-méthyl]-1,3-diméthyl-3H-imidazol-1-ium ;
- le chlorure de 4-{2-[2-(2-amino-5-hydroxy-phényl)-acétylamino]-éthyl}-1,3-diméthyl-3H-imidazol-1-ium ;
- le chlorure de 4-[(5-amino-2-hydroxy-benzylcarbamoyl)-méthyl]-1,3-diméthyl-3H-imidazol-1-ium ; et leurs sels d'addition.

10. Composition selon la revendication 9, où les paraphénylènediamines ou paraaminophénols cationiques sont choisies parmi :
- le chlorure de [2-(2,5-diamino-phénoxy)-éthyl]-diéthyl-méthyl-ammonium ;
- le chlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [4-(4-amino-phénylamino)-pentyl]-diéthyl-(2-hydroxyéthyl)-ammonium ;
- le chlorure de 3-[3-(4-amino-phénylamino)-propyl]-1-méthyl-3H-imidazol-1-ium ;
- le chlorure de1-{3-(2,5-diamino-phénoxy)-propyl]-3-méthyt-3H-imidazol-1-ium ;
- le chlorure, de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 3-[3-(4-amino-2-fluoro-phénylamino)-propyl]-1-méthyl-3H-imidazol-1-ium ;
- le chlorure de 3-[3-(4-amino-2-cyano-phénylamino)-propyl]-1-méthyl-3H-imidazol-1-ium et leurs sels d'addition.

11. Composition selon la revendication 8, où les paraphénylènediamines ou les para-aminophénols dibenzéniques à groupement cationique sont choisies parmi :
- le dichlorure de 1,3-bis-1 {3{3'[(4"-amino-3"-méthyl-aniline)-N-propyl]}-3H-imidazol-1-ium}-propane ;
- le dichlorure de 1,3-bis-1{3{3'[(4"-amino-2"-méthyl-aniline)-N-propyl]}-3H-imidazol-1-ium}-propane ;
- le dichlorure de 1,3-bis-1 {3{3'[(4"-amino-aniline)-N-propyl]}-3H-imidazol-1-ium}-propane ;
- le dichlorure de 1,3-bis-1 {3{3'[(4"-amino-aniline)-N-propyl]}-3H-imidazol-1-ium}-2-propanol ;
- le dibromure de N₁,N₃-bis-[3-N(4'-amino-aniline)-propyl]-1,1,3,3-tétraméthyl-diammonium 1-3-propane ;
- le dichlorure de 1,4-bis-1{3[3-(2,5-diamino-phénoxy)-propyl]-3H-imidazol-1-ium}- butane ;
- le monochlorure de 1,3-bis-[3-(2,5-diamino-phénoxy)-propyl]-3H-imidazol-1-ium ;
- le dibromure de N₁,N₄-bis-[3-N-méthyl-N-(4'-amino-aniline)-éthyl]-1,1,4,4-tétraméthyl-diammonium 1-3-propane ;
- le dichlorure de 1,4-bis-1-[3-(5-amino-2-hydroxy-benzyl)-3H-imidazol-1 ium]-butane ;
- le dibromure de 1,3-bis-{[2-(4-amino-aniline)-propyl]-1,1,3,3-tétraméthyl-diammonium-propane ;
- le dichlorure de 1,3-bis-{[4-(4-amino-aniline)-pentyl]-1,1,3,3-tétraméthyl-diammonium-propane ;
- le monochlorure de [4-(4-amino-phénylamino)-pentyl]-(5-amino-2-hydroxybenzyl)-diéthyl-ammonium ;
- le monochlorure de [2-(4-amino-phénylamino)-propyl]-(5-amino-2-hydroxybenzyl)-diméthyl-ammonium ;
- le dichlorure de 1,3-bis-1-{3-[3-(2,5-diamino-phénoxy)-propyl]-3H-imidazol-1-ium}-propane ;
- le dichlorure de 1,3-bis-1{3{3'[(4"-amino-aniline)-N-propyl]}-3H-imidazol-1-ium}-propane ;
- le dichlorure de 1,3-bis-1{4{4'(4-[3-(4"-amino-phénylamino)-propyl]}-1,3-diméthyl-3H-imidazol-1-ium}-propane,
- le dichlorure de 1,3-bis-1 {4{4'(4-[3-(4"-amino-2"-méthyl-aniline)-propyl] }-1,3-diméthyl-3H-imidazol-1-ium}-propane ;
- le monochlorure de 4-[2-(2,5-diamino-phénoxy)-éthyl]-3-[3-(2,5-diamino-phénoxy)-propyl]-1-méthyl-3-imidazol-1-ium ;
- le monochlorure de 4-[2-(2,5-diamino-phénoxy)-éthyl]-1-[3-(2,5-diamino-phénoxy)-propyl]-3-méthyl-3-imidazol-1-ium ;
et leurs sels d'addition.

12. Composition selon la revendication 11, où les paraphénylènediamines ou paraaminophénols cationiques dibenzéniques sont choisies parmi :
- le dichlorure de 1,3-bis-1-{3-{3'-[(4"-amino-aniline)-N-propyl]}-3H-imidazol-1-ium}-propane ;
- le monochlorure de 1,3-bis-[3-(2,5-diamino-phénoxy)-propyl]-3H-imidazol-1-ium ;
- le dibromure de N₁,N₄-bis-[3-N-méthyl-N-(4'-amino-aniline)-éthyl]-1,1,4,4-tétraméthyl-diammonium 1-3-propane ;
- le dichlorure de 1,4-bis-1[3-(5-amino-2-hydroxy-benzyl)-3H-imidazol-1-ium]-butane ;
- le dichlorure de 1,3-bis-1{3{3'[(4"-amino-aniline)-N-propyl]}-3H-imidazol-1-ium}-propane et leurs sels d'addition .

13. Composition selon la revendication 8, où les orthophénylènediamines monobenzéniques à groupement cationique sont choisies parmi :
- le monochlorure de {2-[2-amino-phénylamino]-éthyl}-triméthyl-ammonium,
- le monochlorure de [2-(2-amino-5-chloro-phénylamino)-éthyl]-triméthyl-ammonium ;
- le monochlorure de [2-(2-amino-6-chloro-phénylamino)-éthyl]-triméthyl-ammonium ;
- le monochlorure de [2-(2-amino-4-chloro-phénylamino)-éthyl]-triméthyl-ammonium ;
- le monochlorure de {2-[2-amino-4-chloro-5-(2-hydroxyéthoxy)-phénylamino]-éthyl}-triméthyl-ammonium ;
- le monochlorure de [2-(2-amino-5-méthoxy-phénylamino)-éthyl]-triméthyl-ammonium ;
- le monobromure de [2-(2-amino-phénylamino)-éthyl]-(2-hydroxyethyl)-diméthyl-ammonium ;
- le monochlorure de 4-[2-(2-amino-phénylamino)-éthyl]-4-méthyl-morpholin-4-ium ;
- le monochlorure de 1-[2-(2-amino-phénylamino)-éthyl]-1-éthyl-pipéridinium ;
- le monochlorure de 1-[2-(2-amino-phénylamino)-éthyl]-1,4-diméthyl-pipérazin-1-ium ;
- le dichlorure de 4-[2-(1-méthyl-pipéridinium)-éthoxy]-N2-[2-(1-méthyl-pipéridinium)-éthyl]-benzène-1,2-diamine ;
- le monochlorure de 1-[2-(2-amino-5-méthylsulfanyl-phénylamino)-éthyl]-1-méthyl-pipéridinium ;
- le monochlorure de 1-[2-(2-amino-phénylamino)-éthyl]-1-méthyl-pyrrolidinium ;
- le monochlorure de [3-(2-amino-phénylamino)-propyl]-diéthyl-méthyl-ammonium ;
- le dichlorure de N,N'-bis-[2-(1-méthyl-pipéridinium)-éthyl]-benzène-1,2-diamine ;
- le monochlorure de [2-(2-amino-4-méthyl-phénylamino)-éthyl]-triméthyl-
- le monochlorure de 3-[3-(2-amino-phénylamino)-propyl]-1-méthyl-3H-imidazol-1-ium ;
- le monochlorure de 3-[2-(2-amino-phénylamino)-éthyl]-1-méthyl-3H-imidazol-1-ium ;
- le dichlorure de 4-[2-(1-méthyl-3H-imidazol-1-ium)-éthoxy]-N2-[2-(1-méthyl-3H-imidazol-1-ium)-éthyl]-benzène-1,2-diamine ;
- le monochlorure de 3-[2-(2-amino-4-méthyl-phénylamino)-éthyl]-1-éthyl-3H-imidazol-1-ium ;
- le dichlorure de 3-[3-(2-amino-phénylamino)-propyl]-1-(3-triméthyl-ammonium-2-hydroxy-propyl)-3H-imidazol-1-ium ;
- le monobromure de 3-[3-(2-amino-phénylamino)-propyl]-1-(2-hydroxy-éthyl)-3H-imidazol-1-ium
- le monochlorure de 3-{[2-(2-amino-phénylamino)-éthylcarbamoyl]-méthyl}-1-méthyl-3H-imidazol-1-ium ;
- le monochlorure de 1-[2-(2-amino-4-chloro-phénylamino)-éthyl]-pyridinium ;
- le monochlorure de 3-[2-(2-amino-5-méthoxy-phénylamino)-éthyl]-1-méthyl-3H-imidazol-1-ium ;
- le monochlorure de 3-[2-(2-amino-5-méthylsulfanyl-phénylamino)-éthyl]-1-méthyl-3H-imidazol-1-ium ;
et leurs sels d'addition.

14. Composition selon la revendication 13, où les orthophénylènediamines monobenzéniques à groupement cationique sont choisies parmi :
- le chlorure de {2-[2-amino-phénylamino]-éthyl}-triméthyl-ammonium :
- le chlorure de 3-[3-(2-amino-phénylamino)-propyl]-1-méthyl-3H-imidazol-1-ium
et leurs sels d'addition.

15. Composition selon la revendication 8, où les où les orthophénylènediamines dibenzéniques à groupement cationique sont choisies parmi :
- le dibromure de 1,3-bis-{3-{3-[(2-amino-aniline)-N-propyl]}-3H-imidazol-1-ium}-propane,
- le dibromure de N₁,N₃-bis-[3-N-(2-amino-aniline)-propyl]-1,1,3,3-tétraméthyl-diammonium 1-3-propane,
- le dichlorure de 1,4-bis-{3-{2-[(2-amino-aniline)-N-éthyl]}-3H-imidazol-1-ium}-butane,
- le monochlorure de 1-[2-(2-amino-aniline)-éthyl]-3-[3-(2-amino-aniline)-propyl]-3H-imidazol-1-ium, et leurs sels d'addition.

16. Composition selon la revendication 15, où les où les orthophénylènediamines dibenzéniques à groupement cationique sont choisies parmi :
- le dibromure de 1,3-bis-1-{3-{3-[(2-amino-aniline)-N-propyl)}-3H-imidazol-1-ium}-propane,
le dibromure de N1,N3-bis-[3-N-(2-amino-aniline)-propyl]-1,1,3,3-tétraméthyl-diammonium 1-3-propane, et leurs sels d'addition.

17. Composition selon la revendication 8, où les bases hétérocycliques cationiques du type pyrazole, sont choisies parmi :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-pyrazol-1-yl)-éthyl]-1-méthyl-3H-imidazol-1-ium;
- le chlorure de [2-(4,5-diamino-3-méthyl-pyrazol-1-yl)-éthyl]-triméthyl-ammonium ;
- le chlorure de [2-(4,5-diamino-pyrazol-1-yl)-éthyl]-triméthyl-ammonium ;
- le chlorure de [2-(4-amino-5-hydroxy-pyrazol-1-yl)-éthyl]-triméthyl-ammonium ;
- le chlorure de [2-(4-amino-5-hydroxy-3-méthyl-pyrazol-1-yl)-éthyl]-triméthyl-ammonium ;
- le chlorure de 3-[2-(4-amino-5-hydroxy-3-méthyl-pyrazol-1-yl)-éthyl]-1-méthyl-3H-imidazol-1-ium ;
- le chlorure de 3-[2-(4-amino-5-hydroxy-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium,
et leurs sels d'addition et leurs possibles formes tautomères.

18. Composition selon la revendication 17, où les bases hétérocycliques cationiques du type pyrazole, sont choisies parmi :
- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium ;
et leurs sels d'addition avec un acide ou avec une base, et leurs possibles formes tautomères.

19. Composition selon la revendication 8, où les bases hétérocycliques du type pyrazolo-[1,5-a]-pyrimidine, sont choisies parmi :
- le monobromure de 1-[3-(3-amino-5,6-diméthyl-pyrazolo-[1,5-a]-pyrimidin-7-ylamino)-propyl]-3-méthyl- 3H-imidazol-1-ium ,
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le chlorure de 3-[(3-amino-pyrazolo[1,5-a]pyrimidin-7-ylcarbamoyl)-méthyl]-1-méthyl-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le méthyl sulfate de 2-[(3-amino-pyrazolo[1,5-a]pyrimidin-7-ylamino)-méthyl]-1,3-diméthyl-3H-imidazol-1-ium,
- le méthyl sulfate de 3-[(3-amino-pyrazolo[1,5-a]pyrimidin-7-ylamino)-méthyl]-1-méthyl-pyridinium,
- le méthyl sulfate de 3-[(3-amino-pyrazolo[1,5-a]pyrimidin-7-ylamino)-méthyl]-1-méthyl-pyridinium,
- le méthyl sulfate de 2-(3,7-diamino-5-méthyl-pyrazolo[1,5-a]pyrimidin-6-ylméthyl)-1,3-diméthyl-3H-imidazol-1-ium,
- le méthyl sulfate de 2-(3-amino-7-hydroxy-5-méthyl-pyrazolo[1,5-a]pyrimidin-6-ylméthyl)-1,3-diméthyl-3H-imidazol-1-ium,
- le méthyl sulfate de 2-(3,7-diamino-pyrazolo[1,5-a]pyrimidin-2-yl)-1-méthyl-pyridinium,
- le chlorure de [3-(3-amino-5-méthyl-pyrazolo[1,5-a]pyrimidin-7-ylamino)-propyl]-triméthyl-ammonium,
- le méthyl sulfate de [3-(3-amino-5-méthyl-pyrazolo[1,5-a]pyrimidin-7-ylamino)-propyl]-triméthyl-ammonium,
- le chlorure de 1-[3-(3-amino-5-méthyl-pyrazolo[1,5-a]pyrimidin-7-ylamino)-propyl]-1-méthyl-pipéridinium,
- le méthyl sulfate de 1-[3-(3-amino-5-méthyl-pyrazolo[1,5-a]pyrimidin-7-ylamino)-propyl]-1-méthyl-pipéridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition.

20. Composition selon la revendication 19, où les bases hétérocycliques du type pyrazolo-[1,5-a]-pyrimidine, sont choisies parmi :
- le monobromure de 1-[3-(3-amino-5,6-diméthyl-pyrazolo-[1,5-a]-pyrimidin-7-ylamino)-propyl]-3-méthyl- 3H-imidazol-1-ium ,
- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le chlorure de 3-(3-amino-7-hydroxy-5-méthyl-pyrazolo[1,5-a]pyrimidin-6-ylméthyl)-1-méthyl-pyridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,
et leurs sels d'addition.

21. Composition selon l'une quelconque des revendications 1 à 20, dans laquelle le coupleur est choisi parmi les métaphénylènediamines, les méta-aminophénols, les métadiphénols, les coupleurs naphtaléniques, les coupleurs hétérocycliques et leurs sels d'addition.

22. Composition selon la revendication 21, dans laquelle la quantité de chacun des coupleurs est comprise entre 0,001 et 10 % en poids environ du poids total de la composition tinctoriale.

23. Composition selon l'une quelconque des revendications 1 à 22, comprenant au moins une base d'oxydation additionnelle non-cationique choisie parmi les paraphénylènediamines, les bis-phénylalkylènediamines, les para-aminophénols, les ortho-aminophénols, les bases hétérocycliques autre que les bases d'oxydation de formule (I) et leurs sels d'addition.

24. Composition selon l'une quelconque des revendications 1 à 23, dans laquelle la quantité de chacune des bases d'oxydation est comprise entre 0,001 à 10 % en poids environ du poids total de la composition tinctoriale.

25. Composition selon l'une quelconque des revendications 1 à 24, comprenant un agent oxydant.

26. Composition selon la revendication 24, dans laquelle l'agent oxydant est choisi parmi le peroxyde d'hydrogène, le peroxyde d'urée, les bromates de métaux alcalins, les persels, les peracides et les enzymes oxydases.

27. Procédé de teinture d'oxydation des fibres kératiniques **caractérisé en ce qu'**on applique sur les fibres au moins une composition telle que définie à l'une quelconque des revendications 1 à 24, et qu'on révèle la couleur à l'aide d'un agent oxydant.

28. Procédé selon la revendication 27, dans lequel l'agent oxydant est choisi parmi le peroxyde d'hydrogène, le peroxyde d'urée, les bromates de métaux alcalins, les persels, les peracides et les enzymes oxydases.

29. Procédé selon l'une des revendications 27 ou 28, dans lequel l'agent oxydant est mélangé au moment de l'emploi à la composition telle que définie selon l'une quelconque des revendications 1 à 23.

30. Procédé selon l'une quelconque des revendications 27 ou 28, dans lequel l'agent oxydant est appliqué sur les fibres sous forme de composition oxydante simultanément ou séquentiellement à la composition telle que définie selon l'une quelconque des revendications 1 à 24.

31. Dispositif à plusieurs compartiments dans lequel un premier compartiment contient une composition tinctoriale telle que définie à l'une quelconque des revendications 1 à 24 et un deuxième compartiment contient une composition oxydante.

32. Utilisation de la composition tinctoriale définie aux revendications 1 à 25 pour la teinture de fibres kératiniques.

## Patentansprüche

1. Farbmittelzusammensetzung, die in einem zum Färben geeigneten Träger enthält:
- mindestens ein 4,5-Diaminopyrazol der Formel (I) oder ein entsprechendes Additionssalz dieser Verbindungen (Oxidationsbase): 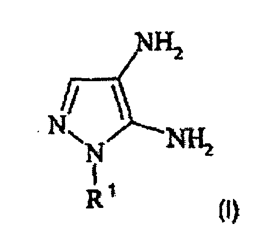
worin R¹ eine C₁₋₆-Alkylgruppe bedeutet, die mit einer oder mehreren Gruppen OR² substituiert ist, wobei R² eine C₁₋₆-Alkylgruppe ist;
- mindestens eine Monobenzol-Oxidationsbase, Dibenzol-Oxidationsbase oder heterocyclische Oxidationsbase, die mindestens eine kationische Gruppe Z aufweist, die unter einer aliphatischen Kette ausgewählt ist, die gegebenenfalls einen gesättigten oder ungesättigten Ring enthält; wobei die Gruppe Z an mindestens einen Benzolring oder den Heterocyclus der Oxidationsbase direkt gebunden ist oder an mindestens eine Aminofunktion des Benzolrings oder des Heterocyclus gebunden ist; oder eines ihrer Additionssalze, und
- mindestens einen Kuppler.

2. Zusammensetzung nach Anspruch 1, wobei die Oxidationsbase der Formel (I) so vorliegt, dass R¹ eine C₁₋₄-Alkylgruppe bedeutet, die mit OR² substituiert ist, wobei R₂ eine C₁₋₄-Alkylgruppe ist.

3. Zusammensetzung nach Anspruch 2, wobei die Oxidationsbase der Formel (I) das 4,5-Diamino-1-(2'-methoxyethyl)-pyrazol ist.

4. Zusammensetzung nach einem der Ansprüche 1 bis 3, wobei Z unter den ungesättigten kationischen Gruppen der folgenden Formeln (II) und (III) und den gesättigten kationischen Gruppen der folgenden Formel (IV) ausgewählt ist: 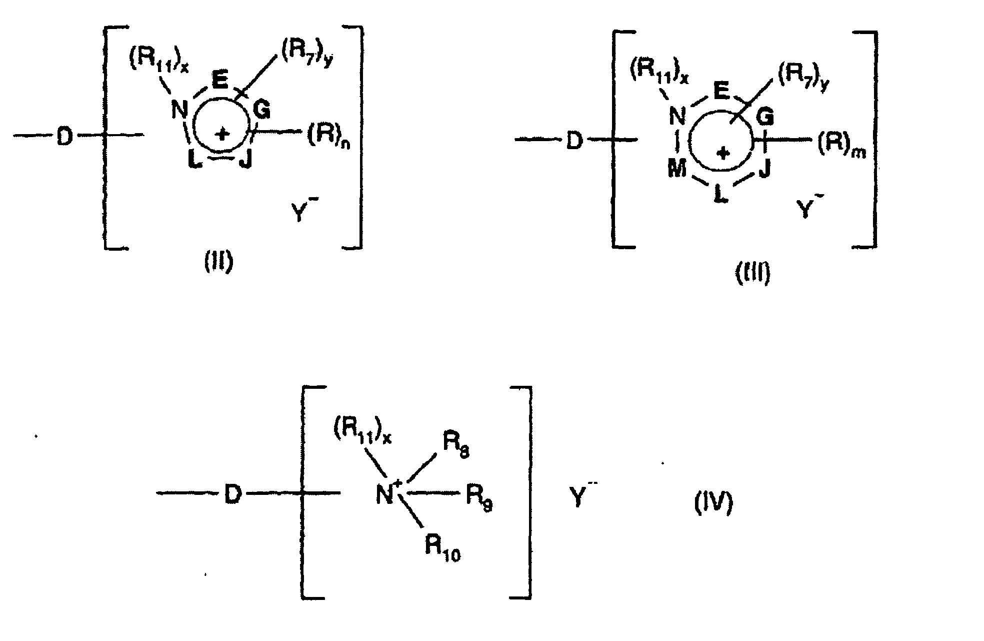 worin bedeuten:
- D eine Verbindungsgruppe, die eine geradkettige oder verzweigte Alkylenkette mit vorzugsweise 1 bis 14 Kohlenstoffatomen bedeutet, die mit einem oder mehreren Heteroatomen, wie Sauerstoff, Schwefel oder Stickstoff unterbrochen und mit einer und mehreren Hydroxygruppen oder C₁₋₆-Alkoxygruppen substituiert sein kann und eine oder mehrere Ketofunktionen aufweisen kann;
- die Atome E, G, J, L und M, die gleich oder verschieden sind, ein Kohlenstoffatom, ein Sauerstoffatom, ein Schwefelatom oder ein Stickstoffatom;
- n 0 oder eine ganze Zahl von 1 bis 4;
- m 0 oder eine ganze Zahl von 1 bis 5;
- die Gruppen R, R₈ bis R₁₁, die gleich oder verschieden sind, ein Halogenatom, Hydroxy, Amino, Cyano, Amido, SO₃X, Aryl, eine Gruppe Z₁, OZ₁, NHZ₁, NZ₁Z₂; wobei zwei der Gruppen R₈ bis Rio einen gesättigten 5- oder 6-gliedrigen Heterocyclus bilden können, der mit einem oder mehreren Heteroatomen unterbrochen sein und einen oder mehrere Substituenten tragen kann, die unter den Gruppen, Hydroxy, Amino, Halogen, Cyano, Amido, CO₂X, C₁₋₆-Alkyl, C₁₋₆-Alkoxy, C₁₋₆-Monoalkylamino, C₁₋₆-Dialkylamino ausgewählt sind;
- die Gruppen Z₁ und Z₂, die gleich oder verschieden sind, eine geradkettige oder verzweigte C₁₋₁₄-Kohlenwasserstoffkette, die durch ein oder mehrere Heteroatome unterbrochen sein kann und einen oder mehrere Substituenten aufweisen kann, die unter den Gruppen Hydroxy, Amino, Amido, Halogen, Cyano, CO₂X, C₁₋₆-Alkyl, C₁₋₆-Alkoxy, C₁₋₆-Monoalkylamino, C₁₋₆-Dialkylamino ausgewählt sind;
- X Wasserstoff, ein Erdalkalimetall oder Alkalimetall, das Ammoniumion, C₁₋₆-Alkyl oder einen von einem organischen Amin stammenden Rest;
- Y- ein einwertiges oder zweiwertiges Anion, das von einer organischen oder anorganischen Säure stammt, wobei Y- vorzugsweise unter Halogenen, wie Chlor, Brom, Fluor, Iod, Hydrogensulfat, Alkyl(C₁₋₆)sulfat, wie beispielsweise Methylsulfat oder Ethylsulfat, ausgewählt ist;
- x und y 0 oder 1; mit den folgenden Maßgaben:
in den ungesättigten kationischen Gruppen der Formel (II):
- die Verbindungsgruppe D ist an das Stickstoffatom gebunden, wenn x = 0,
- die Verbindungsgruppe D ist an eines der Atome E, G, J oder L gebunden, wenn x = 1,
- y kann nur den Wert 1 annehmen, wenn:
1) die Atome E, G, J und L gleichzeitig ein Kohlenstoffatom bedeuten und die Gruppe R₇ von dem Stickstoffatom des ungesättigten Rings getragen wird; oder
2) mindestens eines der Atome E, G, J und L ein Stickstoffatom bedeutet, an das die Gruppe R₇ gebunden ist;
in den ungesättigten kationischen Gruppen der Formel (III):
- die Verbindungsgruppe D an das Stickstoffatom gebunden ist, wenn x = 0,
- die Verbindungsgruppe D ist an eines der Atome E, G, J oder L oder M gebunden, wenn x = 1,
- y kann nur den Wert 1 annehmen, wenn mindestens eines der Atome E, G, J, L und M ein zweiwertiges Atom bedeutet und die Gruppe R₇ von dem Stickstoffatom des ungesättigten Rings getragen wird;
in den ungesättigten kationischen Gruppen der Formel (IV):
- die Verbindungsgruppe D ist an das Stickstoffatom gebunden, das die Gruppen R₈ bis Rio trägt, wenn x = 0,
- zwei der Gruppen R₈ bis R₁₀ bilden gemeinsam mit dem Stickstoffatom, an das sie gebunden sind, einen gesättigten, 5- oder 6-gliedrigen Ring, der oben definiert wurde, und die Verbindungsgruppe D wird von einem Kohlenstoffatom des gesättigten Rings getragen, wenn x = 1;

5. Zusammensetzung nach Anspruch 4, wobei die ungesättigten Ringe Z der Formel (II) unter den Ringen Pyrrol, Imidazol, Pyrazol, Oxazol, Thiazol und Triazol ausgewählt sind.

6. Zusammensetzung nach Anspruch 4, wobei die ungesättigten Ringe Z der Formel (III) unter den Ringen Pyridin, Pyrimidin, Pyrazin, Oxazin und Triazin ausgewählt sind.

7. Zusammensetzung nach Anspruch 4, wobei die gesättigten Ringe Z der Formel (IV) unter den Ringen Pyrrolidin, Piperidin, Piperazin und Morpholin ausgewählt sind.

8. Zusammensetzung nach einem der Ansprüche 1 bis 7, wobei die kationischen Oxidationsbasen ausgewählt sind unter:
(i) p-Phenylendiaminen oder p-Aminophenolen auf Monobenzolbasis;
(ii) p-Phenylendiaminen oder p-Aminophenolen auf Dibenzolbasis;
(iii) o-Phenylendiaminen auf Monobenzolbasis;
(iv) o-Phenylendiaminen auf Dibenzolbasis;
(v) heterocyclischen Basen vom Pyrazoltyp; und
(vi) heterocyclischen Basen vom Typ Pyrazolo-[1,5-a]-pyrimidin.

9. Zusammensetzung nach Anspruch 8, wobei die p-Phenylendiamine oder p-Aminophenole vom Monobenzoltyp mit kationischer Gruppe ausgewählt sind unter:
- [2-(2,5-Diaminophenoxy)-ethyl]-diethylmethylammonium-chlorid;
- N,N-Bis-(trimethylammoniumpropyl)-4-aminoanilindichlorid;
- [4-(4-Aminophenylamino)-pentyl]-diethylmethylammonium-chlorid;
- [4-(4-Aminophenylamino)-pentyl]-diethyl-(2-hydroxyethyl)-ammoniumchlorid;
- [2-(4-Aminophenylamino)-ethyl]-diethylmethylammonium-chlorid;
- {2-[(4-Aminophenyl)-methylamino]-ethyl)-trimethylammoniumchlorid;
- [3-(4-Aminophenylamino)-propyl]-trimethylammoniumchlorid;
- [2-(4-Aminophenylamino)-propyl]-trimethylammoniumchlorid;
- [4-(4-Amino-2-methylphenylamino)-pentyl]-diethylmethyl-ammoniumchlorid;
- [4-(4-Amino-3-methylphenylamino)-pentyl]-diethylmethyl-ammoniumchlorid;
- 1-{[5-Amino-2-(2-hydroxyethylamino)-phenylcarbamoyl]-methyl}-1,4-dimethylpiperazin-1-ium-chlorid;
- 1-[2-(4-Aminophenylamino)-ethyl]-3-methyl-3H-imidazol-1-ium-bromid;
- 1-[3-(2,5-Diaminophenoxy)-propyl]-3-methyl-3H-imidazol-1-ium-chlorid;
- 3-[3-(4-Aminophenylamino)-propyl]-1-methyl-3H-imidazol-1-ium-chlorid;
- 3-[3-(4-Amino-3-methylphenylamino)-propyl]-1-methyl-3H-imidazol-1-ium-chlorid;
- 3-[3-(4-Amino-2-methylphenylamino)-propyl]-1-methyl-3H-imidazol-1-ium-chlorid;
- 3-[3-(4-Amino-2-fluorphenylamino)-propyl]-1-methyl-3H-imidazol-1-ium-chlorid;
- 3-[3-(4-Amino-2-cyanophenylamino)-propyl]-1-methyl-3H-imidazol-1-ium-chlorid;
- 1-[2-(4-Amino-2-methoxyphenylamino)-ethyl]-3-methyl-3H-imidazol-1-ium-chlorid;
- 1-(5-Amino-2-hydroxybenzyl)-3-methyl-3H-imidazol-1-ium-chlorid;
- 1-(5-Amino-2-hydroxybenzyl)-2-methyl-2H-pyrazol-1-ium-chlorid;
- 1-(2-(2,5-Diaminophenyl)-ethyl]-3-methyl-3H-imidazol-1-ium-chlorid;
- 3-[2-(2,5-Diaminophenyl)-ethyl]-1-methyl-3H-imidazol-1-ium-chlorid;
- 1-{2-[(4-aminophenyl)-ethylamino]-ethyl]-3-methyl-3H-imidazol-1-ium-chlorid;
- N,N-Bis[2-(3-methyl-3H-imidazol-1-ium)-ethyl]-4-aminoanilin-dichlorid;
- 3-[2-(4-Aminophenylamino)-butyl]-1-methyl-3H-imidazol-1-ium-chlorid;
- 1-{[5-Amino-2-(2-hydroxyethylamino)-phenylcarbamoyl]-methyl}-3-methyl-3H-imidazol-1-ium-chlorid;
- 4-[2-(2,5-Diaminophenoxy)-ethyl]-1,3-dimethyl-3H-imidazol-1-ium-bromid;
- 2-(2,5-Diaminophenoxymethyl)-1,3-dimethyl-3H-imidazol-1-ium-chlorid;
- 4-[3-(4-Aminophenylamino)-propyl]-1,3-dimethyl-3H-imidazol-1-ium-chlorid;
- 4-[3-(4-Amino-3-methylphenylamino)-propyl]-1,3-dimethyl-3H-imidazol-1-ium-chlorid;
- 4-[(2,5-Diaminophenylcarbamoyl)-methyl]-1,3-dimethyl-3H-imidazol-1-ium-chlorid;
- 4-{2-[2-(2-Amino-5-hydroxyphenyl)-acetylamino]-ethyl}-1,3-dimethyl-3H-imidazol-1-ium-chlorid;
- 4-[(5-Amino-2-hydroxybenzylcarbamoyl)-methyl]-1,3-dimethyl-3H-imidazol-1-iumchlorid; und deren Additionssalzen.

10. Zusammensetzung nach Anspruch 9, wobei die kationischen p-Phenylendiamine oder p-Aminophenole ausgewählt sind unter:
- [2-(2,5-Diaminophenoxy)-ethyl]-diethylmethylammonium-chlorid;
- N,N-Bis-(trimethylammoniumpropyl)-4-aminoanilinchlorid;
- [4-(4-Aminophenylamino)-pentyl]-diethylmethylammonium-chlorid;
- [2-(4-Aminophenylamino)-ethyl]-diethylmethylammonium-chlorid;
- {2-[(4-Aminophenyl)-methylamino]-ethyl)-trimethylammoniumchlorid;
- [3-(4-Aminophenylamino)-propyl]-trimethylammoniumchlorid;
- [4-(4-Aminophenylamino)-pentyl]-diethyl-(2-hydroxyethyl)-ammoniumchlorid;
- 3-[3-(4-Aminophenylamino)-propyl]-1-methyl-3H-imidazol-1-ium-chlorid;
- 1-[3-(2,5-Diaminophenoxy)-propyl]-3-methyl-3H-imidazol-1-ium-chlorid;
- 3-[3-(4-Amino-3-methylphenylamino)-propyl]-1-methyl-3H-imidazol-1-ium-chlorid;
- 3-[3-(4-Amino-2-methylphenylamino)-propyl]-1-methyl-3H-imidazol-1-ium-chlorid;
- 1-[2-(4-Amino-2-methoxyphenylamino)-ethyl]-3-methyl-3H-imidazol-1-ium-chlorid;
- 3-[3-(4-Amino-2-fluorphenylamino)-propyl]-1-methyl-3H-imidazol-1-ium-chlorid;
- 3-[3-(4-Amino-2-cyanophenylamino)-propyl]-1-methyl-3H-imidazol-1-ium-chlorid; und deren Additionssalzen.

11. Zusammensetzung nach Anspruch 8, wobei die p-Phenylendiamine oder p-Aminophenole auf Dibenzolbasis mit kationischer Gruppe ausgewählt sind unter:
- 1,3-Bis-1{3{3'[(4"-amino-3"-methylanilin)-N-propyl]}-3H-imidazol-1-ium}-propandichlorid;
- 1,3-Bis-1{3{3'[(4"-amino-3"-methylanilin)-N-propyl]}-3H-imidazol-1-ium}-propandichlorid;
- 1,3-Bis-1{3{3'[(4"-aminoanilin)-N-propyl]}-3H-imidazol-1-ium}-propandichlorid;
- 1,3-Bis-1{3{3'[(4"-aminoanilin)-N-propyl]}-3H-imidazol-1-ium}-2-propanoldichlorid;
- N₁,N₃-Bis[3-N(4'-aminoanilin)-propyl]-1,1,3,3-tetramethyl-diammonium-1-3-propandibromid;
- 1,4-Bis-1{3[3-(2,5-diaminophenoxy)-propyl]-3H-imidazol-1-ium}-butandichlorid;
- 1,3-Bis[3-(2,5-diaminophenoxy)-propyl]-3H-imidazol-1-ium}-monochlorid;
- N₁,N₄-Bis[3-N-methyl-N-(4'-aminoanilin)-ethyl]-1,1,4,4-tetramethyldiammonium-1-3-propandibromid;
- 1,4-Bis-1-[3-(5-amino-2-hydroxybenzyl)-3H-imidazol-1-ium]-butandichlorid;
- 1,3-Bis{[2-(4-aminoanilin)-propyl]-1,1,3,3-tetramethyl-diammoniumpropandibromid;
- 1,3-Bis{[4-(4-aminoanilin)-pentyl]-1,1,3,3-tetramethyl-diammoniumpropandichlorid;
- [4-(4-Aminophenylamino)-pentyl]-(5-amino-2-hydroxybenzyl)-diethylammoniummonochlorid
- [2-(4-Aminophenylamino)-propyl]-(5-amino-2-hydroxybenzyl)-dimethylammoniummonochlorid;
- 1,3-Bis-1-{3-[3-(2,5-diaminophenoxy)-propyl]-3H-imidazol-1-ium}-propandichlorid;
- 1,3-Bis-1-{3{3'[(4"-aminoanilin)-N-propyl]-3H-imidazol-1-ium}-propandichlorid;
- 1,3-Bis-1-{4{4'(4-[3-(4"-aminophenylamino)-propyl}}-1,3-dimethyl-3H-imidazol-1-ium}-propandichlorid;
- 1,3-Bis-1-{4{4'(4-[3-(4"-amino-2"-methylanilin)-propyl}}-1,3-dimethyl-3H-imidazol-1-ium}-propandichlorid;
- 4-[2-(2,5-Diaminophenoxy)-ethyl]-3-[3-(2,5-diaminophenoxy)-propyl]-1-methyl-3-imidazol-1-ium-monochlorid;
- 4-[2-(2,5-Diaminophenoxy)-ethyl]-1-[3-(2,5-diaminophenoxy)-propyl]-3-methyl-3-imidazol-1-ium-monochlorid;
und deren Additionssalzen.

12. Zusammensetzung nach Anspruch 11, wobei die kationischen p-Phenylendiamine oder p-Aminophenole auf Dibenzolbasis ausgewählt sind unter:
- 1,3-Bis-1-{3-{3'[(4"-aminoanilin)-N-propyl]}-3H-imidazol-1-ium}-propandichlorid;
- 1,3-Bis[3-(2,5-diaminophenoxy)-propyl]-3H-imidazol-1-ium}-monochlorid;
- N₁,N₄-Bis[3-N-methyl-N-(4'-aminoanilin)-ethyl]-1,1,4,4-tetramethyldiammonium-1-3-propandibromid;
- 1,4-Bis-1[3-(5-amino-2-hydroxybenzyl)-3H-imidazol-1-ium]-butandichlorid;
- 1,3-Bis-1{3{3'[(4"-aminoanilin)-N-propyl]}-3H-imidazol-1-ium]-propandichlorid;
und deren Additionssalzen.

13. Zusammensetzung nach Anspruch 8, wobei die o-Phenylendiamine auf Monobenzolbasis mit kationischer Gruppe ausgewählt sind unter:
- {2-[2-Aminophenylamino]-ethyl}-trimethylammoniummonochlorid;
- [2-(2-Amino-5-chlorphenylamino)-ethyl]-trimethylammoniummonochlorid;
- [2-(2-Amino-6-chlorphenylamino)-ethyl]-trimethylammoniummonochlorid;
- [2-(2-Amino-4-chlorphenylamino)-ethyl]-trimethylammoniummonochlorid;
- {2-[2-Amino-4-chlor-5-(2-hydroxyethoxy)-phenylamino]-ethyl}-trimethylammoniummonochlorid;
- [2-(2-Amino-5-methoxyphenylamino)-ethyl]-trimethylammoniummonochlorid;
- [2-(2-Aminophenylamino]-ethyl]-(2-hydroxyethyl)-dimethylammoniummonobromid;
- 4-[2-(2-Aminophenylamino)-ethyl]-4-methylmorpholin-4-ium-monochlorid;
- 1-[2-(2-Aminophenylamino)-ethyl]-1-ethylpiperidinium-monochlorid;
- 1-[2-(2-Aminophenylamino)-ethyl]-1,4-dimethylpiperazin-1-ium-monochlorid;
- 4-[2-(1-Methylpiperidinium)-ethoxy]-N2-[2-(1-methylpiperidinium)-ethyl]-benzol-1,2-diamindichlorid;
- 1-[2-(2-Amino-5-methylsulfanylphenylamino)-ethyl]-1-methyl-piperidiniummonochlorid;
- 1-[2-(2-Aminophenylamino)-ethyl]-1-methylpyrrolidinium-monochlorid;
- [3-(2-Aminophenylamino)-propyl]-diethylmethylammonium-monochlorid;
- N,N'-Bis[2-(1-methylpiperidinium)-ethyl]-benzol-1,2-diamin-dichlorid;
- [2-(2-Amino-4-methylphenylamino)-ethyl]-trimethylmonochlorid;
- 3-[3-(2-Aminophenylamino)-propyl]-1-methyl-3H-imidazol-1-ium-monochlorid;
- 3-[2-(2-Aminophenylamino)-ethyl]-1-methyl-3H-imidazol-1-ium-monochlorid;
- 4-[2-(1-Methyl-3H-imidazol-1-ium)-ethoxy]-N2-[2-(1-methyl-3H-imidazol-1-ium)-ethyl]-benzol-1,2-diamindichlorid;
- 3-[2-(2-Amino-4-methylphenylamino)-ethyl]-1-ethyl-3H-imidazol-1-ium-monochlorid;
- 3-[3-(2-Aminophenylamino)-propyl]-1-(3-trimethylammonium-2-hydroxypropyl)-3H-imidazol-1-ium-dichlorid;
- 3-[3-(2-Aminophenylamino)-propyl]-1-(2-hydroxyethyl)-3H-imidazol-1-ium-monobromid;
- 3-{[2-(2-Aminophenylamino)-ethylcarbmnoyl]-methyl}-1-methyl-3H-imidazol-1-ium-monochlorid;
- 1-[2-(2-Amino-4-chlorphenylamino)-ethyl]-pyridiniummonochlorid;
- 3-[2-(2-Amino-5-methoxyphenylamino)-ethyl]-1-methyl-3H-imidazol-1-ium-monochlorid;
- 3-[2-(2-Amino-5-methylsulfanylphenylamino)-ethyl]-1-methyl-3H-imidazol-1-ium-monochlorid;
und deren Additionssalzen.

14. Zusammensetzung nach Anspruch 13, wobei die o-Phenylendiamine auf Monobenzolbasis mit kationischer Gruppe ausgewählt sind unter:
- {2-[2-Aminophenylamino]-ethyl}-trimethylammoniumchlorid;
- 3-[3-(2-Aminophenylaminon)-propyl]-1-methyl-3H-imidazol-1-ium-chlorid und deren Additionssalze.

15. Zusammensetzung nach Anspruch 8, wobei die o-Phenylendiamine auf Dibenzolbasis mit kationischer Gruppe ausgewählt sind unter:
- 1,3-Bis{3-{3-[(2-aminoanilin)-N-propyl]}-3H-imidazol-1-ium}-propandibromid,
- N₁,N₃-Bis[3-N-(2-aminoanilin)-propyl]-1,1,3,3-tetramethyl-diammonium-1-3-propandibromid,
- 1,4-Bis{3-{2-[(2-aminoanilin)-N-ethyl]}-3H-imidazol-1-ium}-butandichlorid,
- 1-[2-(2-Aminoanilin)-ethyl]-3-[3-(2-aminoanilin)-propyl]-3H-imidazol-1-ium-monochlorid und deren Additionssalzen.

16. Zusammensetzung nach Anspruch 15, wobei die o-Phenylendiamine auf Monobenzolbasis mit kationischer Gruppe ausgewählt sind unter:
- 1,3-Bis-1-{3-{3-[(2-aminoanilin)-N-propyl]}-3H-imidazol-1-ium}-propandibromid,
- N1,N3-Bis[3-N-(2-aminoanilin)-propyl]-1,1,3,3-tetramethyldiammonium-1-3-propandibromid und deren Additionssalzen.

17. Zusammensetzung nach Anspruch 8, wobei die kationischen heterocyclischen Basen vom Pyrazoltyp ausgewählt sind unter:
- [3-(4-Amino-2,5-dimethyl-2H-pyrazol-3-yl-amino)-propyl]-trimethylammoniumchlorid;
- [3-(4-Amino-2,5-dimethyl-2H-pyrazol-3-yl-amino)-propyl]-(2-hydroxyethyl)-dimethylammoniumchlorid;
- 3-[3-(4-Amino-2,5-dimethyl-2H-pyrazol-3-yl-amino)-propyl]-1-(2-hydroxyethyl)-3H-imidazol-1-iumchlorid;
- 3- [(4-Amino-2H-pyrazol-3-yl-carbamoyl)-methyl]-1-methyl-3H-imidazol-1-ium-chlorid;
- 3-[2-(4,5-Diamino-3-methylpyrazol-1-yl)-ethyl]-1-methyl-3H-imidazol-1-ium-chlorid;
- 3-[2-(4,5-Diaminopyrazol-1-yl)-ethyl]-1-methyl-3H-imidazol-1-ium-chlorid;
- [2-(4,5-Diamino-3-methylpyrazol-1-yl)-ethyl]-trimethylammoniumchlorid;
- [2-(4,5-Diaminopyrazol-1-yl)-ethyl]-trimethylammoniumchlorid;
- [2-(4-Amino-5-hydroxypyrazol-1-yl)-ethyl]-trimethylammoniumchlorid;
- [2-(4-Amino-5-hydroxy-3-methylpyrazol-1-yl)-ethyl]-trimethylammoniumchlorid;
- 3-[2-(4-Amino-5-hydroxy-3-methylpyrazol-1-yl)-ethyl]-1-methyl-3 H-imidazol-1-ium-chlorid;
- 3-[2-(4-Amino-5-hydroxypyrazol-1-yl)-ethyl]-1-methyl-3H-imidazol-1-ium-chlorid;
- 3-[2-(4,5-Diamino-1-methyl-1H-pyrazol-3-yl)-ethyl]-1-methyl-3H-imidazol-1-ium-chlorid,
und deren Additionssalzen und deren möglichen tautomeren Formen.

18. Zusammensetzung nach Anspruch 17, wobei die kationischen heterocyclischen Basen vom Pyrazoltyp ausgewählt sind unter:
- [3-(4-Amino-2,5-dimethyl-2H-pyrazol-3-yl-amino)-propyl]-trimethylammoniumchlorid;
- (3-(4-Amino-2,5-dimethyl-2H-pyrazol-3-yl-amino)-propyl]-(2-hydroxyethyl)-dimethylammoniumchlorid;
- 3-[3-(4-Amino-2,5-dimethyl-2H-pyrazol-3-yl-amino)-propyl]-1-(2-hydroxyethyl)-3H-imidazol-1-iumchlorid;
- 3-[(4-Amino-2H-pyrazol-3-yl-carbamoyl)-methyl]-1-methyl-3H-imidazol-1-ium-chlorid;
- 3-[2-(4,5-Diamino-3-methylpyrazol-1-yl)-ethyl]-1-methyl-3H-imidazol-1-ium-chlorid;
- 3-[2-(4,5-Diamino-1-methyl-1H-pyrazol-3-yl)-ethyl]-1-methyl-3H-imidazol-1-ium-chlorid;
und deren Additionssalzen mit einer Säure oder mit einer Base und ihren möglichen tautomeren Formen.

19. Zusammensetzung nach Anspruch 8, wobei die heterocyclischen Basen vom Pyrazolo-[1,5-a]-pyrimidin-Typ ausgewählt sind unter:
- 1-[3-(3-Amino-5,6-dimethylpyrazolo-[1,5-a]-pyrimidin-7-yl-amino)-propyl]-3-methyl-3H-imidazol-1-ium-monobromid,
- 3-[3-(3-Amino-5-methyl-pyrazolo[1,5-a]pyrimidin-7-yl-amino)-propyl]-1-(2-hydroxyethyl)-3H-imidazol-1-ium-chlorid,
- 3-[(3-Aminopyrazolo[1,5-a]pyrimidin-7-yl-carbamoyl)-methyl]-1-methyl-3H-imidazol-1-ium-chlorid,
- 3-(3-Amino-7-hydroxy-5-methylpyrazolo[1,5-a]-pyrimidin-6-yl-methyl)-1-methylpyridiniummethylsulfat,
- 3-(3-Amino-7-hydroxy-5-methylpyrazolo[1,5-a]pyrimidin-6-yl-methyl)-1-(2-hydroxyethyl)-pyridiniumchlorid,
- 2-[(3-Aminopyrazolo[1,5-a]pyrimidin-7-yl-amino)-methyl]-1,3-dimethyl-3H-imidazol-1-ium-methylsulfat,
- 3-[(3-Aminopyrazolo[1,5-a]pyrimidin-7-yl-amino)-methyl]-1-methylpyridiniummethylsulfat,
- 3-[(3-Aminopyrazolo[1,5-a]pyrimidin-7-yl-amino)-methyl]-1-methylpyridiniummethylsulfat,
- 2-(3,7-Diamino-5-methylpyrazolo[1,5-a]pyrimidin-6-yl-methyl)-1,3-dimethyl-3H-imidazol-1-ium-methylsulfat,
- 2-(3-Amino-7-hydroxy-5-methylpyrazolo[1,5-a]pyrimidin-6-yl-methyl)-1,3-dimethyl-3H-imidazol-1-ium-methylsulfat,
- 2-(3,7-Diaminopyrazolo[1,5-a]pyrimidin-2-yl)-1-methylpyridiniummethylsulfat,
- [3-(3-Amino-5-methylpyrazolo[1,5-a]pyrimidin-7-yl-amino)-propyl]-trimethylammoniumchlorid,
- [3-(3-Amino-5-methylpyrazolo[1,5-a]pyrimidin-7-yl-amino)-propyl]-trimethylammoniummethylsulfat,
- 1-[3-(3-Amino-5-methylpyrazolo[1,5-a]pyrimidin-7-yl-amino)-propyl]-1-methylpiperidiniumchlorid,
- 1-[3-(3-Amino-5-methylpyrazolo[1,5-a]pyrimidin-7-yl-amino)-propyl]-1-methylpiperidiniummethylsulfat,
- 4-[3-(3-Amino-5-methylpyrazolo[1,5-a]pyrimidin-7-yl-amino)-propyl]-4-methylmorpholin-4-ium-chlorid,
- 4-[3-(3-Amino-5-methylpyrazolo[1,5-a]pyrimidin-7-yl-amino)-propyl]-4-methylmorpholin-4-ium-methylsulfat,
und deren Additionssalzen.

20. Zusammensetzung nach Anspruch 19, wobei die heterocyclischen Basen vom Pyrazolo-[1,5-a]-pyrimidin-Typ ausgewählt sind unter:
- 1-[3-(3-Amino-5,6-dimethylpyrazolo-[1,5-a]-pyrimidin-7-yl-amino)-propyl]-3-methyl-3H-imidazol-1-ium-monobromid,
- 3-[3-(3-Amino-5-methylpyrazolo[1,5-a]pyrimidin-7-yl-amino)-propyl]-1-(2-hydroxyethyl)-3H-imidazol-1-ium-chlorid,
- 3-(3-Amino-7-hydroxy-5-methylpyrazolo[1,5-a]-pyrimidin-6-yl-methyl)-1-methylpyridiniummethylsulfat,
- 3-(3-Amino-7-hydroxy-5-methylpyrazolo[1,5-a]pyrimidin-6-yl-methyl)-1-(2-hydroxyethyl)-pyridiniumchlorid,
- 3-(3-Amino-7-hydroxy-5-methylpyrazolo[1,5-a]pyrimidin-6-yl-methyl)-1-methylpyridiniumchlorid,
- 4-[3-(3-Amino-5-methylpyrazolo[1,5-a]pyrimidin-7-yl-amino)-propyl]-4-methylmorpholin-4-ium-chlorid,
- 4-[3-(3-Amino-5-methylpyrazolo[1,5-a]pyrimidin-7-yl-amino)-propyl]-4-methylmorpholin-4-ium-methylsulfat,
und deren Additionssalzen.

21. Zusammensetzung nach einem der Ansprüche 1 bis 20, wobei der Kuppler unter den m-Phenylendiaminen, m-Aminophenolen, m-Dihydroxybenzolen, Naphthalinkupplern, heterocyclischen Kupplern und deren Additionssalzen ausgewählt ist.

22. Zusammensetzung nach Anspruch 21, wobei der Mengenanteil jedes Kupplers im Bereich von 0,001 bis 10 Gew.-% des Gesamtgewichts der Farbmittelzusammensetzung liegt.

23. Zusammensetzung nach einem der Ansprüche 1 bis 22, die mindestens eine zusätzliche nicht kationische Oxidationsbase enthält, die unter den p-Phenylendiaminen, Bisphenylalkylendiaminen, p-Aminophenolen, o-Aminophenolen, und heterocyclischen Basen, die von den Oxidationsbasen der Formel (I) verschieden sind, und deren Additionssalzen ausgewählt ist.

24. Zusammensetzung nach einem der Ansprüche 1 bis 23, wobei der Mengenanteil jeder Oxidationsbase im Bereich von etwa 0,001 bis 10 Gew.-% des Gesamtgewichts der Farbmittelzusammensetzung liegt.

25. Zusammensetzung nach einem der Ansprüche 1 bis 24, die ein Oxidationsmittel enthält.

26. Zusammensetzung nach Anspruch 24, wobei das Oxidationsmittel unter Wasserstoffperoxid, Harnstoffperoxid, Alkalimetallbromaten, Salzen von Persäuren, Persäuren und Oxidasen ausgewählt ist.

27. Verfahren zum oxidativen Färben von Keratinfasern, **dadurch gekennzeichnet, dass** auf die Fasern mindestens eine Zusammensetzung nach einem der Ansprüche 1 bis 24 aufgetragen und die Farbe mit einem Oxidationsmittel entwickelt wird.

28. Verfahren nach Anspruch 27, wobei das Oxidationsmittel unter Wasserstoffperoxid, Harnstoffperoxid, Alkalimetallbromaten, Salzen von Persäuren, Persäuren und Oxidasen ausgewählt ist.

29. Verfahren nach einem der Ansprüche 27 oder 28, wobei das Oxidationsmittel bei der Anwendung mit der Zusammensetzung nach einem der Ansprüche 1 bis 23 vermischt wird.

30. Verfahren nach einem der Ansprüche 27 oder 28, wobei das Oxidationsmittel auf die Fasern in Form einer oxidierenden Zusammensetzung aufgebracht wird, die gleichzeitig mit oder nach der Zusammensetzung nach einem der Ansprüche 1 bis 24 aufgetragen wird.

31. Vorrichtung mit mehreren Abteilungen, wobei eine erste Abteilung eine Farbmittelzusammensetzung nach einem der Ansprüche 1 bis 24 enthält, und eine zweite Zusammensetzung eine oxidierende Zusammensetzung enthält.

32. Verwendung der Farbmittelzusammensetzung nach einem der Ansprüche 1 bis 25 zum Färben von Keratinfasern.

## Claims

1. Dye composition comprising, in a suitable dyeing medium:
- at least one 4,5-diaminopyrazole oxidation base of formula (I) or the corresponding addition salts 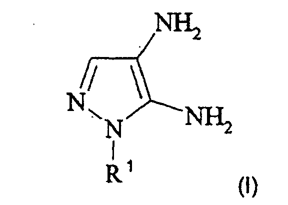 in which R¹ is a C₁-C₆ alkyl radical substituted with one or more radicals OR², R² being a C₁-C₆ alkyl radical;
- at least one monobenzenic, dibenzenic or heterocyclic oxidation base comprising at least one cationic group Z chosen from an aliphatic chain optionally bearing a saturated or unsaturated ring; the said group Z being linked directly to at least one benzenic ring or to the heterocycle of the said oxidation base or alternatively linked to at least one amine function via the said benzenic ring or the said heterocycle; or one of the addition salts thereof, and
- at least one coupler.

2. Composition according to Claim 1, in which the oxidation base of formula (I) is such that R¹ represents a C₁-C₄ alkyl radical substituted with a radical OR², R² being a C₁-C₄ alkyl radical.

3. Composition according to Claim 2, in which the oxidation base of formula (I) is 4,5-diamino-1-(2'-methoxyethyl)pyrazole.

4. Composition according to any one of Claims 1 to 3, in which Z is chosen from the unsaturated cationic groups of formulae (II) and (III) below, and the saturated cationic groups of formula (IV) below: 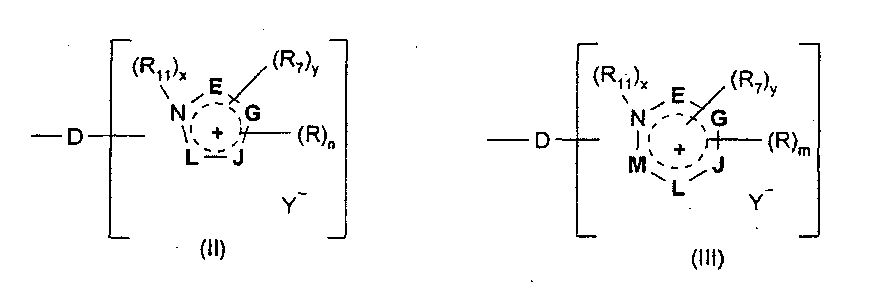 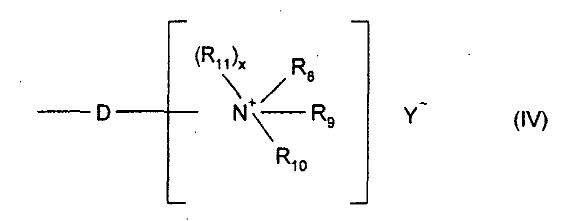 in which:
- D is a linker arm that represents a linear or branched alkylene chain preferably containing from 1 to 14 carbon atoms, which may be interrupted with one or more hetero atoms such as oxygen, sulphur or nitrogen atoms, and which may be substituted with one or more hydroxyl or C₁-C₆ alkoxy radicals, and which may bear one or more ketone functions;
- the ring members E, G, J, L and M, which may be identical or different, represent a carbon, oxygen, sulphur or nitrogen atom;
- n is an integer ranging from 0 to 4;
- m is an integer ranging from 0 to 5;
- the radicals R and R₈ to R₁₁, which may be identical or different, represent a halogen atom, a hydroxyl radical, an amino group, a cyano group, an amido group, SO₃X, an aryl, a radical Z₁, OZ₁, NHZ₁ or NZ₁Z₂; two of the radicals R₈ to R₁₀ may form a 5- or 6-membered saturated heterocycle, which may be interrupted with one or more hetero atoms and which may comprise one or more substituents chosen from hydroxyl, amino, halogen, cyano, amido and CO₂X groups, a C₁-C₆ alkyl radical, a C₁-C₆ alkoxy radical, a C₁-C₆ monoalkylamino radical or a C₁-C₆ dialkylamino radical;
- Z₁ and Z₂, which may be identical or different, represent a linear or branched C₁-C₁₄ hydrocarbon-based chain, which may be interrupted with one or more hetero atoms and which may comprise one or more substituents chosen from hydroxyl, amino, amido, halogen, cyano and CO₂X groups, a C₁-C₆ alkyl radical, a C₁-C₆ alkoxy radical, a C₁-C₆ monoalkylamino radical or a C₁-C₆ dialkylamino radical;
- X denotes hydrogen, an alkali metal or alkaline-earth metal residue, the ammonium ion, a C₁-C₆ alkyl radical or a residue derived from an organic amine;
- Y⁻ represents a monovalent or divalent anion derived from a mineral or organic acid and is preferably chosen from a halogen atom such as chlorine, bromine, fluorine or iodine, a hydrogen sulphate, or a (C₁-C₆)alkyl sulphate such as, for example, a methyl sulphate or an ethyl sulphate.
- x and y are integers equal to 0 or 1; with the following conditions:
in the unsaturated cationic groups of formula (II):
- when x = 0, the linker arm D is attached to the nitrogen atom,
- when x = 1, the linker arm D is attached to one of the ring members E, G, J or L,
- y can take the value 1 only:
1) when the ring members E, G, J and L simultaneously represent a carbon atom, and when the radical R₇ is borne by the nitrogen atom of the unsaturated ring; or alternatively
2) when at least one of the ring members E, G, J and L represents a nitrogen atom to which the radical R₇ is attached;
in the unsaturated cationic groups of formula (III):
- when x = 0, the linker arm D is attached to the nitrogen atom,
- when x = 1, the linker arm D is attached to one of the ring members E, G, J, L or M,
- y can take the value 1 only when at least one of the ring members E, G, J, L and M represents a divalent atom, and when the radical R₇ is borne by the nitrogen atom of the unsaturated ring;
in the cationic groups of formula (IV):
- when x = 0, then the linker arm is attached to the nitrogen atom bearing the radicals R₈ to R₁₀,
- when x = 1, then two of the radicals R₈ to R₁₀ form, together with the nitrogen atom to which they are attached, a 5- or 6-membered saturated ring as defined above, and the linker arm D is borne by a carbon atom of the said saturated ring.

5. Composition according to Claim 4, in which the unsaturated rings Z of formula (II) are chosen from pyrrole, imidazole, pyrazole, oxazole, thiazole and triazole rings.

6. Composition according to Claim 4, in which the unsaturated rings Z of formula (III) are chosen from pyridine, pyrimidine, pyrazine, oxazine and triazine rings.

7. Composition according to Claim 4, in which the saturated rings Z of formula (IV) are chosen from pyrrolidine, piperidine, piperazine and morpholine rings.

8. Composition according to any one of Claims 1 to 7, in which the cationic oxidation bases are chosen from:
(i) monobenzenic para-phenylenediamines or para-aminophenols;
(ii) dibenzenic para-phenylenediamines or para-aminophenols;
(iii) monobenzenic ortho-phenylenediamines;
(iv) dibenzenic ortho-phenylenediamines;
(v) heterocyclic bases of the pyrazole type;
(vi) heterocyclic bases of the pyrazolo[1,5-a]-pyrimidine type.

9. Composition according to Claim 8, in which the monobenzenic para-phenylenediamines or para-aminophenols containing a cationic group are chosen from:
- [2-(2,5-diaminophenoxy)ethyl]diethylmethylammonium chloride;
- N,N-bis(trimethylammoniumpropyl)-4-aminoaniline dichloride;
- [4-(4-aminophenylamino)pentyl]diethylmethylammonium chloride;
- [4-(4-aminophenylamino)pentyl]diethyl-(2-hydroxyethyl)ammonium chloride;
- [2-(4-aminophenylamino)ethyl]diethylmethylammonium chloride;
- {2-[(4-aminophenyl)methylamino]ethyl}trimethylammonium chloride;
- [3-(4-aminophenylamino)propyl]trimethylammonium chloride;
- [2-(4-aminophenylamino)propyl]trimethylammonium chloride;
- [4-(4-amino-2-methylphenylamino)pentyl]diethyl-methylammonium chloride;
- [4-(4-amino-3-methylphenylamino)pentyl]diethyl-methylammonium chloride;
- 1-{[5-amino-2-(2-hydroxyethylamino)phenylcarbamoyl]-methyl}-1,4-dimethylpiperazin-1-ium chloride;
- 1-[2-(4-aminophenylamino)ethyl]-3-methyl-3H-imidazol-1-ium bromide;
- 1-[3-(2,5-diaminophenoxy)propyl]-3-methyl-3H-imidazol-1-ium chloride;
- 3-[3-(4-aminophenylamino}propyl]-1-methyl-3H-imidazol-1-ium chloride;
- 3-[3-(4-amino-3-methylphenylamino)propyl]-1-methyl-3H-imidazol-1-ium chloride;
- 3-[3-(4-amino-2-methylphenylamino)propyl]-1-methyl-3H-imidazol-1-ium chloride;
- 3-[3-(4-amino-2-fluorophenylamino)propyl]-1-methyl-3H-imidazol-1-ium chloride;
- 3-[3-(4-amino-2-cyanophenylamino)propyl]-2-methyl-3H-imidazol-1-ium chloride;
- 1-[2-(4-amino-2-methoxyphenylamino)ethyl]-3-methyl-3H-imidazol-1-ium chloride;
- 1-(5-amino-2-hydroxybenzyl)-3-methyl-3H-imidazol-1-ium chloride;
- 1-(5-amino-2-hydroxybenzyl)-2-methyl-2H-pyrazol-1-ium chloride;
- 1-[2-(2,5-diaminophenyl)ethyl]-3-methyl-3H-imidazol-1-ium chloride;
- 3-[2-(2,5-diaminophenyl)ethyl]-2-methyl-3H-imidazol-1-ium chloride;
- 1-{2-[(4-aminophenyl)ethylamino]ethyl}-3-methyl-3H-imidazol-1-ium chloride;
- N,N-bis[2-(3-methyl-3H-imidazol-1-ium)ethyl]-4-aminoaniline dichloride;
- 3-[2-(4-aminophenylamino)butyl]-1-methyl-3H-imidazol-1-ium chloride;
- 1-{[5-amino-2-(2-hydroxyethylamino)phenylcarbamoyl]-methyl}-3-methyl-3H-imidazol-1-ium chloride;
- 4-[2-(2,5-diaminophenoxy)ethyl]-1,3-dimethyl-3H-imidazol-1-ium bromide;
- 2-(2,5-diaminophenoxymethyl)-1,3-dimethyl-3H-imidazol-1-ium chloride;
- 4-[3-(4-aminophenylamino)propyl]-1,3-dimethyl-3H-imidazol-1-ium chloride;
- 4-[3-(4-amino-3-methylphenylamino)propyl]-1,3-dimethyl-3H-imidazol-1-ium chloride;
- 4-[(2,5-diaminophenylcarbamoyl)methyl]-1,3-dimethyl-3H-imidazol-1-ium chloride;
- 4-{2-[2-(2-amino-5-hydroxyphenyl)acetylamino]ethyl}-1,3-dimethyl-3H-imidazol-1-ium chloride;
- 4-[(5-amino-2-hydroxybenzylcarbamoyl)methyl]-1,3-dimethyl-3H-imidazol-1-ium chloride; and the addition salts thereof.

10. Composition according to Claim 9, in which the cationic para-phenylenediamines or para-aminophenols are chosen from:
- [2-(2,5-diaminophenoxy)ethyl]diethylmethylammonium chloride;
- N,N-bis(trimethylammoniumpropyl)-4-aminoaniline chloride;
- [4-(4-aminophenylamino)pentyl]diethylmethylammonium chloride;
- [2-(4-aminophenylamino)ethyl]diethylmethylammonium chloride;
- {2-[(4-aminophenyl)methylamino]ethyl}trimethylammonium chloride;
- [3-(4-aminophenylamino)propyl]trimethylammonium chloride;
- [4-(4-aminophenylamino)pentyl]diethyl-(2-hydroxyethyl)ammonium chloride;
- 3-[3-(4-aminophenylamino)propyl]-1-methyl-3H-imidazol-1-ium chloride;
- 1-[3-(2,5-diaminophenoxy)propyl]-3-methyl-3H-imidazol-1-ium chloride;
- 3-[3-(4-amino-3-methylphenylamino)propyl]-1-methyl-3H-imidazol-1-ium chloride;
- 3-[3-(4-amino-2-methylphenylamino)propyl]-1-methyl-3H-imidazol-1-ium chloride;
- 1-[2-(4-amino-2-methoxyphenylamino)ethyl]-3-methyl-3H-imidazol-1-ium chloride;
- 3-[3-(4-amino-2-fluorophenylamino)propyl]-1-methyl-3H-imidazol-1-ium chloride;
- 3-[3-(4-amino-2-cyanophenylamino)propyl]-1-methyl-3H-imidazol-1-ium chloride and the addition salts thereof.

11. Composition according to Claim 8, in which the dibenzenic para-phenylenediamines or para-aminophenols containing a cationic group are chosen from:
- 1,3-bis-1{3{3'[(4"-amino-3"-methylaniline)-N-propyl]}-3H-imidazol-1-ium}propane dichloride;
- 1,3-bis-1{3{3'[(4"-amino-2"-methylaniline)-N-propyl]}-3H-imidazol-1-ium}propane dichloride;
- 1,3-bis-1{3{3'[(4"-aminoaniline)-N-propyl]}-3H-imidazol-1-ium}propane dichloride;
- 1,3-bis-1{3{3'[(4"-aminoaniline)-N-propyl]}-3H-imidazol-1-ium}-2-propanol dichloride;
- N₁,N₃-bis-[3-N-(4'-aminoaniline)propyl]-1,1,3,3-tetramethyl-1,3-diammoniumpropane dibromide;
- 1,4-bis-1{3[3-(2,5-diaminophenoxy)propyl]-3H-imidazol-1-ium}butane dichloride;
- 1,3-bis[3-(2,5-diaminophenoxy)propyl]-3H-imidazol-1-ium monochloride;
- N₁,N₄-bis-[3-N-methyl-N-(4'-aminoaniline)ethyl]-1,1,4,4-tetramethyl-1,3-diammoniumpropane dibromide;
- 1,4-bis-1-[3-(5-amino-2-hydroxybenzyl)-3H-imidazol-1-ium]butane dichloride;
- 1,3-bis-{[2-(4-aminoaniline)propyl]-1,1,3,3-tetramethyldiammoniumpropane dibromide;
- 1,3-bis-{[4-(4-aminoaniline)pentyl]-1,1,3,3-tetramethyldiammoniumpropane dichloride;
- [4-(4-aminophenylamino)pentyl]-(5-amino-2-hydroxybenzyl)diethylammonium monochloride;
- [2-(4-aminophenylamino)propyl]-(5-amino-2-hydroxybenzyl)dimethylammonium monochloride;
- 1,3-bis-1-{3-[3-(2,5-diaminophenoxy)propyl]-3H-imidazol-1-ium}propane dichloride;
- 1,3-bis-1-{3{3'[(4"-aminoaniline)-N-propyl]}-3H-imidazol-1-ium}propane dichloride;
- 1,3-bis-1-{4{4'(4-[3-(4"-aminophenylamino)propyl]}-1,3-dimethyl-3H-imidazol-1-ium}propane dichloride;
- 1,3-bis-1-{4{4'(4-[3-(4"-amino-2"-methylaniline)propyl]}-1,3-dimethyl-3H-imidazol-1-ium}propane dichloride;
- 4-[2-(2,5-diaminophenoxy)ethyl]-3-[3-(2,5-diaminophenoxy)propyl]-1-methyl-3-imidazol-1-ium monochloride;
- 4-[2-(2,5-diaminophenoxy)ethyl]-1-[3-(2,5-diaminophenoxy)propyl]-3-methyl-3-imidazol-1-ium monochloride; and the addition salts thereof.

12. Composition according to Claim 11, in which the dibenzenic cationic para-phenylenediamines or para-aminophenols are chosen from:
- 1,3-bis-1-{3{3'-[(4"-aminoaniline)-N-propyl]}-3H-imidazol-1-ium}propane dichloride;
- 1,3-bis[3-(2,5-diaminophenoxy)propyl]-3H-imidazol-1-ium monochloride;
- N₁,N₄-bis-[3-N-methyl-N-(4'-aminoaniline)ethyl]-1,1,4,4-tetramethyl-1,3-diammoniumpropane dibromide;
- 1,4-bis-1-[3-(5-amino-2-hydroxybenzyl)-3H-imidazol-1-ium]butane dichloride;
- 1,3-bis-1-{3{3'[(4"-aminoaniline)-N-propyl]}-3H-imidazol-1-ium}propane dichloride;
and the addition salts thereof.

13. Composition according to Claim 8, in which the monobenzenic ortho-phenylenediamines containing a cationic group are chosen from:
- {2-[2-aminophenylaminolethyl}trimethylammonium monochloride;
- [2-(2-amino-5-chlorophenylamino)ethyl]trimethylammonium monochloride;
- [2-(2-amino-6-chlorophenylamino)ethyl]trimethylammonium monochloride;
- [2-(2-amino-4-chlorophenylamino)ethyl]trimethylammonium monochloride;
- {2-[2-amino-4-chloro-5-(2-hydroxyethoxy)phenylamino]-ethyl}trimethylammonium monochloride;
- [2-(2-amino-5-methoxyphenylamino)ethyl]trimethylammonium monochloride;
- [2-(2-aminophenylamino)ethyl]-(2-hydroxyethyl)-dimethylammonium monobromide;
- 4-[2-(2-aminophenylamino)ethyl]-4-methylmorpholin-4-ium monochloride;
- 1-[2-(2-aminophenylamino)ethyl]-1-ethylpiperidinium monochloride;
- 1-[2-(2-aminophenylamino)ethyl]-1,4-dimethylpiperazin-1-ium monochloride;
- 4-[2-(1-methylpiperidinium)ethoxy]-N2-[2-(2-methylpiperidinium)ethyl]benzene-1,2-diamine dichloride;
- 1-[2-(2-amino-5-methylsulphanylphenylamino)ethyl]-1-methylpiperidinium monochloride;
- 1-[2-(2-aminophenylamino)ethyl]-1-methylpyrrolidinium monochloride;
- [3-(2-aminophenylamino)propyl]diethylmethylammonium monochloride;
- N,N'-bis-[2-(1-methylpiperidinium)ethyl]benzene-1,2-diamine dichloride;
- [2-(2-amino-4-methylphenylamino)ethyl]trimethyl monochloride;
- 3-[3-(2-aminophenylamino)propyl]-1-methyl-3H-imidazol-1-ium monochloride;
- 3-[2-(2-aminophenylamino)ethyl]-1-methyl-3H-imidazol-1-ium monochloride;
- 4-[2-(2-methyl-3H-imidazol-1-ium)ethoxy]-N2-[2-(1-methyl-3H-imidazol-1-ium)ethyl]benzene-1,2-diamine dichloride;
- 3-[2-(2-amino-4-methylphenylamino)ethyl]-1-ethyl-3H-imidazol-1-ium monochloride;
- 3-[3-(2-aminophenylamino)propyl]-1-(3-trimethylammonium-2-hydroxypropyl)-3H-imidazol-1-ium dichloride;
- 3-[3-(2-aminophenylamino)propyl]-1-(2-hydroxyethyl)-3H-imidazol-1-ium monobromide;
- 3-{[2-(2-aminophenylamino)ethylcarbamoyl]methyl}-1-methyl-3H-imidazol-1-ium monochloride;
- 1-[2-(2-amino-4-chlorophenylamino)ethyl]pyridinium monochloride;
- 3-[2-(2-amino-5-methoxyphenylamino)ethyl]-1-methyl-3H-imidazol-1-ium monochloride;
- 3-[2-(2-amino-5-methylsulphanylphenylamino)ethyl]-1-methyl-3H-imidazol-1-ium monochloride;
and the addition salts thereof.

14. Composition according to Claim 13, in which the monobenzenic ortho-phenylenediamines containing a cationic group are chosen from:
- {2-[2-aminophenylamino]ethyl}trimethylammonium chloride;
- 3-[3-(2-aminophenylamino)propyl]-1-methyl-3H-imidazol-1-ium chloride
and the addition salts thereof.

15. Composition according to Claim 8, in which the dibenzenic ortho-phenylenediamines containing a cationic group are chosen from:
- 1,3-bis-{3-{3-[(2-aminoaniline)-N-propyl]}-3H-imidazol-1-ium}propane dibromide;
- N₁,N₃-bis-[3-N-(2-aminoaniline)propyl]-1,1,3,3-tetramethyl-1,3-diammoniumpropane dibromide;
- 1,4-bis-{3-{2-[(2-aminoaniline)-N-ethyl]}-3H-imidazol-1-ium}butane dichloride;
- 1-[2-(2-aminoaniline)ethyl]-3-[3-(2-aminoaniline)propyl]-3H-imidazol-1-ium monochloride,
and the addition salts thereof.

16. Composition according to Claim 15, in which the dibenzenic ortho-phenylenediamines containing a cationic group are chosen from:
- 1,3-bis-1-{3-{3-[(2-aminoaniline)-N-propyl])-3H-imidazol-1-ium}propane dibromide;
- N₁,N₃-bis[3-N-(2-aminoaniline)propyl]-1,1,3,3-tetramethyl-1,3-diammoniumpropane dibromide, and the addition salts thereof.

17. Composition according to Claim 8, in which the cationic heterocyclic bases of the pyrazole type are chosen from:
- [3-(4-amino-2,5-dimethyl-2H-pyrazol-3-ylamino)propyl]trimethylammonium chloride;
- [3-(4-amino-2,5-dimethyl-2H-pyrazol-3-ylamino)propyl]-(2-hydroxyethyl)dimethylammonium chloride;
- 3-[3-(4-amino-2,5-dimethyl-2H-pyrazol-3-ylamino)propyl]-1-(2-hydroxyethyl)-3H-imidazol-1-ium chloride;
- 3-[(4-amino-2H-pyrazol-3-ylcarbamoyl)methyl]-1-methyl-3H-imidazol-1-ium chloride;
- 3-[2-(4,5-diamino-3-methylpyrazol-1-yl)ethyl]-1-methyl-3H-imidazol-1-ium chloride;
- 3-[2-(4,5-diaminopyrazol-1-yl)ethyl]-1-methyl-3H-imidazol-1-ium chloride;
- [2-(4,5-diamino-3-methylpyrazol-1-yl)ethyl]trimethylammonium chloride;
- [2-(4,5-diaminopyrazol-1-yl)ethyl]trimethylammonium chloride;
- [2-(4-amino-5-hydroxypyrazol-1-yl)ethyl]trimethylammonium chloride;
- [2-(4-amino-5-hydroxy-3-methylpyrazol-1-yl)ethyl]-trimethylammonium chloride;
- 3-[2-(4-amino-5-hydroxy-3-methylpyrazol-1-yl)ethyl]-1-methyl-3H-imidazol-1-ium chloride;
- 3-[2-(4-amino-5-hydroxypyrazol-1-yl)ethyl]-1-methyl-3H-imidazol-1-ium chloride;
- 3-[2-(4,5-diamino-1-methyl-1H-pyrazol-3-yl}ethyl]-1-methyl-3H-imidazol-1-ium chloride,
and the addition salts thereof, and the possible tautomeric forms thereof.

18. Composition according to Claim 17, in which the cationic heterocyclic bases of the pyrazole type are chosen from:
- [3-(4-amino-2,5-dimethyl-2H-pyrazol-3-ylamino)-propyl]trimethylammonium chloride;
- [3-(4-amino-2,5-dimethyl-2H-pyrazol-3-ylamino)-propyl]-(2-hydroxyethyl)dimethylammonium chloride;
- 3-[3-(4-amino-2,5-dimethyl-2H-pyrazol-3-ylamino)-propyl]-1-(2-hydroxyethyl)-3H-imidazol-1-ium chloride;
- 3-[(4-amino-2H-pyrazol-3-ylcarbamoyl)methyl]-1-methyl-3H-imidazol-1-ium chloride;
- 3-[2-(4,5-diamino-3-methylpyrazol-1-yl)ethyl]-1-methyl-3H-imidazol-1-ium chloride;
- 3-[2-(4,5-diamino-1-methyl-1H-pyrazol-3-yl)ethyl]-1-methyl-3H-imidazol-1-ium chloride,
and the addition salts thereof with an acid or with a base, and the possible tautomeric forms thereof.

19. Composition according to Claim 8, in which the heterocyclic bases of the pyrazolo[1,5-a]pyrimidine type are chosen from:
- 1-[3-(3-amino-5,6-dimethylpyrazolo[1,5-a]pyrimidin-7-ylamino)propyl]-3-methyl-3H-imidazol-1-ium monobromide,
- 3-[3-(3-amino-5-methylpyrazolo[1,5-a]pyrimidin-7-ylamino)propyl]-1-(2-hydroxyethyl)-3H-imidazol-1-ium chloride,
- 3-[(3-aminopyrazolo[1,5-a]pyrimidin-7-ylcarbamoyl)-methyl]-1-methyl-3H-imidazol-1-ium chloride,
- 3-(3-amino-7-hydroxy-5-methylpyrazolo[1,5-a]-pyrimidin-6-ylmethyl)-1-methylpyridinium methyl sulphate,
- 3-(3-amino-7-hydroxy-5-methylpyrazolo[1,5-a]-pyrimidin-6-ylmethyl)-1-(2-hydroxyethyl)pyridinium chloride,
- 2-[(3-aminopyrazolo[1,5-a]pyrimidin-7-ylamino)-methyl]-1,3-dimethyl-3H-imidazol-1-ium methyl sulphate,
- 3-[(3-aminopyrazolo[1,5-a]pyrimidin-7-ylamino)-methyl]-1-methylpyridinium methyl sulphate,
- 3-[(3-aminopyrazolo[1,5-a]pyrimidin-7-ylamino)-methyl]-1-methylpyridinium methyl sulphate,
- 2-(3,7-diamino-5-methylpyrazolo[1,5-a]pyrimidin-6-ylmethyl)-1,3-dimethyl-3H-imidazol-1-ium methyl sulphate,
- 2-(3-amino-7-hydroxy-5-methylpyrazolo[1,5-a]-pyrimidin-6-ylmethyl)-1,3-dimethyl-3H-imidazol-1-ium methyl sulphate,
- 2-(3,7-diaminopyrazolo[1,5-a]pyrimidin-2-yl)-1-methylpyridinium methyl sulphate,
- [3-(3-amino-5-methylpyrazolo[1,5-a]pyrimidin-7-ylamino)propyl]trimethylammonium chloride,
- [3-(3-amino-5-methylpyrazolo[1,5-a]pyrimidin-7-ylamino)propyl]trimethylammonium methyl sulphate,
- 1-[3-(3-amino-5-methylpyrazolo[1,5-a]pyrimidin-7-ylamino)propyl]-1-methylpiperidinium chloride,
- 1-[3-(3-amino-5-methylpyrazolo[1,5-a]pyrimidin-7-ylamino)propyl]-1-methylpiperidinium methyl sulphate,
- 4-[3-(3-amino-5-methylpyrazolo[1,5-a]pyrimidin-7-ylamino)propyl]-4-methylmorpholin-4-ium chloride,
- 4-[3-(3-amino-5-methylpyrazolo[1,5-a]pyrimidin-7-ylamino)propyl]-4-methylmorpholin-4-ium methyl sulphate,
and the addition salts thereof.

20. Composition according to Claim 19, in which the heterocyclic bases of the pyrazolo[1,5-a]pyrimidine type are chosen from:
- 1-[3-(3-amino-5,6-dimethylpyrazolo[1,5-a]pyrimidin-7-ylamino)propyl]-3-methyl-3H-imidazol-1-ium monobromide,
- 3-[3-(3-amino-5-methylpyrazolo[1,5-a]pyrimidin-7-ylamino)propyl]-1-(2-hydroxyethyl)-3H-imidazol-2-ium chloride,
- 3-(3-amino-7-hydroxy-5-methylpyrazolo[1,5-a]-pyrimidin-6-ylmethyl)-1-methylpyridinium methyl sulphate,
- 3-(3-amino-7-hydroxy-5-methylpyrazolo[1,5-a]-pyrimidin-6-ylmethyl)-1-(2-hydroxyethyl)pyridinium chloride,
- 3-(3-amino-7-hydroxy-5-methylpyrazolo[1,5-a]-pyrimidin-6-ylmethyl)-1-methylpyridinium chloride,
- 4-[3-(3-amino-5-methylpyrazolo[1,5-a]pyrimidin-7-ylamino)propyl]-4-methylmorpholin-4-ium chloride,
- 4-[3-(3-amino-5-methylpyrazolo[1,5-a]pyrimidin-7-ylamino)propyl]-4-methylmorpholin-4-ium methyl sulphate,
and the addition salts thereof.

21. Composition according to any one of Claims 1 to 20, in which the coupler is chosen from meta-phenylenediamines, meta-aminophenols, meta-diphenols, naphthalenic couplers and heterocyclic couplers, and the addition salts thereof.

22. Composition according to Claim 21, in which the amount of each of the couplers is between 0.001 and 10% by weight approximately relative to the total weight of the dye composition.

23. Composition according to any one of Claims 1 to 22, comprising at least one additional non-cationic oxidation base chosen from para-phenylenediamines, bis(phenyl)alkylenediamines, para-aminophenols, ortho-aminophenols and heterocyclic bases, other than the oxidation bases of formula (I), and the addition salts thereof.

24. Composition according to any one of Claims 1 to 23, in which the amount of each of the oxidation bases is between 0.001% and 10% by weight approximately relative to the total weight of the dye composition.

25. Composition according to any one of Claims 1 to 24, comprising an oxidizing agent.

26. Composition according to Claim 24, in which the oxidizing agent is chosen from hydrogen peroxide, urea peroxide, alkali metal bromates, persalts, peracids and oxidase enzymes.

27. Process for the oxidation dyeing of keratin fibres, **characterized in that** at least one composition as defined in any one of Claims 1 to 24 is applied to the fibres, and **in that** the colour is developed using an oxidizing agent.

28. Process according to Claim 27, in which the oxidizing agent is chosen from hydrogen peroxide, urea peroxide, alkali metal bromates, persalts, peracids and oxidase enzymes.

29. Process according to either of Claims 27 and 28, in which the oxidizing agent is mixed at the time of use with the composition as defined according to any one of Claims 1 to 23.

30. Process according to either of Claims 27 and 28, in which the oxidizing agent is applied to the fibres in the form of an oxidizing composition, simultaneously or sequentially with the composition as defined according to any one of Claims 1 to 24.

31. Multi-compartment device, in which a first compartment contains a dyeing composition as defined in any one of Claims 1 to 24 and a second compartment contains an oxidizing composition.

32. Use of the dye composition as defined in Claims 1 to 25, for dyeing keratin fibres.
